# EUROPEAN PATENT SPECIFICATION

(11) **EP 3 765 628 B1**
(45) Date of publication and mention of the grant of the patent: **14.12.2022**
(21) Application number: 19714722.6
(22) Date of filing: 18.03.2019
(51) Int. Cl.: C12Q 1/25

(54) **LIGASE SCREENING ASSAY**
LIGASE-SCREENING-TEST
ANALYSE DE CRIBLAGE DE LIGASE

(30) Priority: 16.03.2018 GB 201804285
(43) Date of publication of application: 20.01.2021
(73) Proprietor: University of Dundee, Dundee DD1 4HN (GB)
(72) Inventor: VIRDEE, Satpal, Dundee DD5 3SW (GB); PAO, Kuan-Chuan, Douliu City, Yunlin County 640 (TW)
(74) Representative: Marks & Clerk LLP
(86) International application number: PCT/GB2019/050751
(87) International publication number: WO 2019/175609

(56) References cited:
- WO-A1-2016/051174
- ANGELA DÖRR ET AL: "MYCBP2 Is a Guanosine Exchange Factor for Ran Protein and Determines Its Localization in Neurons of Dorsal Root Ganglia", JOURNAL OF BIOLOGICAL CHEMISTRY, vol. 290, no. 42, 16 October 2015 (2015-10-16), pages 25620-25635, XP055584880, US ISSN: 0021-9258, DOI: 10.1074/jbc.M115.646901
- MAEURER C ET AL: "Sphingosine-1-phosphate induced mTOR-activation is mediated by the E3-ubiquitin ligase PAM", CELLULAR SIGNALLING, ELSEVIER SCIENCE LTD, GB, vol. 21, no. 2, 1 February 2009 (2009-02-01), pages 293-300, XP025815294, ISSN: 0898-6568, DOI: 10.1016/J.CELLSIG.2008.10.016 [retrieved on 2008-10-30]
- KATJA K DOVE ET AL: "Molecular insights into RBR E3 ligase ubiquitin transfer mechanisms", EMBO REPORTS, vol. 17, no. 8, 16 June 2016 (2016-06-16), pages 1221-1235, XP55587049, ISSN: 1469-3178, DOI: 10.15252/embr.201642641
- PAO KUAN-CHUAN ET AL: "Activity-based E3 ligase profiling uncovers an E3 ligase with esterification activity", NATURE, MACMILLAN JOURNALS LTD, LONDON, vol. 556, no. 7701, 11 April 2018 (2018-04-11), pages 381-385, XP036759044, ISSN: 0028-0836, DOI: 10.1038/S41586-018-0026-1 [retrieved on 2018-04-11]

## Description

### Field of the invention

The present invention relates to a method for identifying a modulator of MYCBP2 esterification activity towards hydroxyl containing probes. Suitable modulators are identified by modulation of MYCBP2 ubiquitin E3 ligase activity via covalent modification of either of two catalytic cysteines (C4520 and C4572). Hydroxy group-containing small molecules and peptides may be used as proxy substrates for measuring MYCBP2 ligase activity.

### Background

Protein modification with ubiquitin (Ub) and ubiquitin-like modifiers (Ubls) regulates most aspects of eukaryotic biology. Ub/Ubl conjugation is carried out by an enzymatic cascade consisting of E1 activating (E1), E2 conjugating (E2) and E3 ligating (E3) enzymes. Conjugation requires an initial ATP-dependent thioesterification step and up to two subsequent transthioesterification steps via the juxtaposition of catalytic cysteines in E1s, E2s and E3s. Ubiquitination is typically considered a posttranslational modification of lysine residues.

Homologous to E6-AP Carboxy Terminus (HECT) E3s undergo a catalytic cysteine-dependent transthiolation reaction with the E2-ubiquitin covalently linked intermediate to form a covalently linked E3-ubiquitin intermediate. Additionally, RING-between-RING (RBR) E3s have a canonical RING domain that is linked to an ancillary domain. This contains a catalytic cysteine enabling a hybrid RING/HECT mechanism.

The ubiquitin conjugation enzymatic cascade has been implicated in a broad spectrum of diseases. Although numerous different E2 and E3 enzymes and classes have been identified, little is known about some of these enzymes. There therefore remains a need to profile, or further profile the activity of these enzymes.

Dorr et al. (J Biol. Chem., 2015, 290,(42) p25620-25635), discloses a method of identifying whether SUMOylated RanGAP1 inhibits MYCBP2 activity, by contacting MYCBP2 with ubiquitin-E2.

The present invention has been devised with these issues in mind.

### Summary of the invention

The present invention is based on studies by the inventors into the mechanism of action of the E3 ubiquitin ligase MYCBP2. The inventors have surprisingly found that this ligase exhibits esterification activity towards, for example, hydroxy containing substrates. The inventors also believe that transthiolation occurs between E2 conjugating enzyme (E2) and a cysteine residue within MYCBP2. This leads to the intramolecular relay of ubiquitin from the cysteine residue to a further cysteine residue within MYCBP2 and then to its substrate.

Based upon the identification of the mechanism of action, the interaction of the active MYCBP2 protein with a substrate, whether artificial or endogenous, can be studied. This interaction can advantageously be exploited to screen test substances for their effect upon this interaction. It is therefore an object of the present invention to provide a screening assay based upon this interaction. It is a further object of the present invention to identify and/or provide modulators of MYCBP2 for use in therapy and/or prophylaxis, for example in the therapy and/or prophylaxis of axon degeneration-associated injury and disorder.

It will be appreciated that any of the features described herein (including any accompanying claims and drawings), may be combined with any of the below aspects in any combination, unless otherwise indicated.

The present invention is defined in accordance with the claims appended hereto.

For the avoidance of doubt, C4520 is present within the sequence motif GEARCDAEA and C4572 is present within the sequence motif AVFFCFGTT of the full-length MYCBP2 canonical isoform.

Typically the test substance may be a small molecule electrophile, such as an acrylamide, acrylonitrile or an acrylate, when targeting either identified cysteine residue. As a thiol group is present on a cysteine residue which can react with an electrophile, MYCBP2 activity is postulated to be modulated by:
a) covalent modification of C4520 with an electrophilic small molecule ligand; or
b) covalent modification of C4572 with an electrophilic small molecule ligand;

The authors have established that MYCBP2 has unexpectedly high and promiscuous esterification activity towards hydroxy-containing small molecules and peptides. Therefore, the use of small molecule hydroxy compounds as proxy substrates can be used for identifying the electrophilic small molecule ligands. We describe such proxy substrates herein as hydroxy probes.

Without wishing to be bound by theory, the inventors believe that the residues C4520 and C4572 of the full-length native MYCBP2 protein are required for the transthiolation between E2-ubiquitin and MYCBP2, and the subsequent relay of ubiquitin, and so one or more of these residues will be understood to comprise the active site. C4572 operates down-stream and interacts with hydroxy probes. In some embodiments, the active site comprises at least the residue C4520.

MYCBP2 is an 0.5 MDa protein found in humans which contains a C-terminal RING domain. The protein plays a role in axon guidance and synapse formation in the developing nervous system. In mammalian cells, this protein regulates the cAMP and mTOR signaling pathways, and may additionally regulate autophagy. In the context of the present invention it will be understood that MYCBP2, the orthologue, or mutant, or fragment thereof refers to the nucleic acid sequence or expression product thereof, for example DNA, cDNA, mRNA, protein or peptide fragment thereof.

Typically, the MYCBP2, the orthologue, or mutant, or fragment thereof comprises a protein or peptide fragment thereof. Advantageously, this enables the screening of the protein-substrate interaction between the MYCBP2, the orthologue, or mutant, or fragment thereof and the probe.

As used herein, active MYCBP2 will be understood to refer to the enzymatically active MYCBP2 protein.

By "interacting" or "interaction", this will be understood to refer to the association of said probe with an active site of MYCBP2, the orthologue, or mutant, or fragment thereof. The probes described herein may be covalently modified with a ubiquitin molecule.

Hence, the term probe is used herein to refer to a molecule which is capable of interacting with the active site(s) of active MYCBP2 comprising the two catalytic cysteines as defined.

It will be appreciated that the residue numbering of MYCBP2 as used herein is in relation to the native full-length MYCBP2 canonical isoform (Uniprot accession number 075592: hftp://www.uniprot.org/uniprot/O75592), or as described in Pao et al. Nature. 2018 Apr;556(7701):381-385 . Reference to "C" is with reference to the standard amino acid coding. Hence "C" refers to cysteine residue. Subsequent to the first filing of this application, the SWISSPROT entry for MYCBP2 has been amended to include an insertion, which is outside of the catalytic region discussed herein. However, this has the effect of altering sequence numbering by 38 residues. However, we have retained the original numbering as described in the priority application and the Pao et al paper, and the skilled reader can easily identify the relevant cysteine residues and loop region described herein, from the updated sequence numbering, by adding 38 thereto.

Interaction may comprise specific binding of the hydroxyl probe, to the active site of MYCBP2, the orthologue, or mutant, or fragment thereof.

As an enzyme, it will be appreciated that there are endogenous substrates of active MYCBP2. The probe may comprise a native substrate of MYCBP2, i.e. an endogenous substrate known to be capable of interacting with the active site of MYCBP2 *in vivo.* In some embodiments, the probe comprises a non-endogenous substrate of active MYCBP2. By non-endogenous, it will be understood that this includes modified endogenous substrates, as well as substrates, which are not known to interact with MYCBP2 *in vivo* (i.e. artificial substrates).

The probe may be modified or unmodified. For example, the probe may comprise a modified E2, such as an E2-ubiquitin conjugate probe. Examples of such modified probes are provided in WO 2016/051174. Alternatively, in light of our newly presented promiscuous esterification activity demonstrated by MYCBP2, the probe molecule could be an artificial hydroxy containing substrate such as a small molecule or peptide. Activity can be assessed by measuring MYCBP2 and proxy substrate dependent discharge of ubiquitin from upstream E2 conjugating enzyme, or, direct esterification of the proxy substrate with ubiquitin, as described herein.

By "modulator", as used herein, it will be understood that the MYCBP2 modulator increases or decreases the activity of MYCBP2, the orthologue, or mutant, or fragment thereof relative to normal levels (i.e. the level in the absence of the test substance). A modulated level of interaction as compared to the level of interaction in the absence of the test substance may be as a result of a modulated level of activity of MYCBP2.

The modulator may function by disrupting the nucleic acid sequence encoding the MYCBP2, the orthologue, or mutant, or fragment thereof. Alternatively, the modulator may function by disrupting the nucleic acid sequence encoding the active site of the MYCBP2, the orthologue, or mutant, or fragment thereof. For example, step a) may comprise contacting the nucleic acid sequence of MYCBP2, or an orthologue, or mutant, or fragment thereof with a test substance and expressing the protein or peptide product thereof of the contacted nucleic acid sequence. The activity of the expressed protein or peptide product can then be measured. In such embodiments, step c) comprises detecting whether or not the level of interaction of the probe with the expressed protein or peptide product of MYCBP2, the orthologue, or mutant, or fragment thereof is modulated as compared to the level of interaction in the absence of the test substance. In such embodiments, "absence of the test substance" will be understood to refer to wherein the nucleic acid sequence of MYCBP2, an orthologue, or mutant, or fragment thereof is not contacted with the test substance in step a).

In other embodiments, step a) may comprise contacting the MYCBP2, an orthologue, or mutant, or fragment thereof with a test substance, wherein the MYCBP2, the orthologue, or mutant, or fragment thereof comprises a protein or peptide fragment thereof. In such embodiments, step c) comprises detecting whether or not the level of interaction of the probe with the protein or peptide fragment thereof of MYCBP2, the orthologue, or mutant, or fragment thereof is modulated as compared to the level of interaction in the absence of the test substance.

The modulator may be an inhibitor, i.e. it decreases the activity of MYCBP2. In such embodiments, step c) comprises detecting whether or not the level of interaction of the probe with MYCBP2, the orthologue, or mutant, or fragment thereof is reduced as compared to the level of interaction in the absence of the test substance. The activity of MYCBP2 may be reduced by the modulator by at least 10%, at least 20%, at least 30%, at least 40%, at least 50%, at least 60%, at least 70%, at least 80%, at least 90% or 100%.

The inhibitor may function by covalent inactivation of the active site. Alternatively, or in addition to, the inhibitor may function by disrupting binding of the probe to MYCBP2, the orthologue, or mutant, or fragment thereof. In some embodiments the inhibitor functions by disrupting the nucleic acid sequence encoding the MYCBP2, the orthologue, or mutant, or fragment thereof.

Disrupting a nucleic acid sequence may comprise cleavage of the nucleic acid sequence, deletion of a portion of the nucleic acid sequence, degradation of the nucleic acid sequence, destabilization of the nucleic acid sequence and/or insertion of a further nucleic acid sequence into the nucleic acid sequence.

Any suitable substance may be tested as a possible MYCBP2 modulator. It is, however, envisaged, that the method may be used for identifying MYCBP2 modulators for use in therapy and/or prophylaxis, for example in the therapy and/or prophylaxis of neuronal damage, for example, axon degeneration-associated injury and disease. In some embodiments, the test substance comprises an siRNA, an miRNA, a CRISPR/Cas system, a peptide, a protein, an enzyme, a small molecule, an aptamer, an antibody or an antibody fragment (such as a Fab or F(ab')2) fragment, an scFV antibody or any other functional antigen-binding fragment.

As used herein, a "small molecule" is a chemical compound having a molecular weight of no more than 2 Kilo Daltons (kDa). In some embodiments the small molecule has a molecular weight of no more than 1 KDa, or 900 daltons (Da). In some embodiments, the small molecule has a molecular weight of no more than 700 or no more than 500 Da. The small molecule may be an organic compound. Exemplary compounds may include acrylamides, acrylonitriles and acrylates.

In some embodiments the test substance comprises a peptide, a protein, an enzyme, a small molecule, an aptamer, an antibody or an antibody fragment (such as a Fab or F(ab')2) fragment, an scFV antibody or any other functional antigen-binding fragment.

The test substance may comprise an aptamer, an antibody or an antibody fragment (such as a Fab or F(ab')2) fragment, an scFV antibody or any other functional antigen-binding fragment.

In some embodiments, the test substance comprises a CRISPR/Cas system, an siRNA or an miRNA. In such embodiments, step a) comprises contacting the nucleic acid sequence of MYCBP2, an orthologue, or mutant, or fragment thereof with the test substance and expressing the protein or peptide product thereof of the MYCBP2, the orthologue, or mutant, or fragment thereof contacted with the test substance. In such embodiments, step c) comprises detecting whether or not the level of interaction of the probe with the protein or peptide product of step a) is modulated as compared to the level of interaction in the absence of the test substance. It will be appreciated that in such embodiments, a modulator functions by modulating the nucleic acid sequence of MYCBP2, an orthologue, or mutant, or fragment thereof such that the interaction of the probe with the resulting protein or peptide product is modulated.

As the skilled person will be aware, the CRISPR/Cas system is a molecular tool by which a target nucleic acid sequence, typically DNA, can be targeted and cleaved. The target nucleic acid sequence is targeted by a guide RNA (CRISPR RNA or crRNA), the guide RNA forming a duplex with a further small RNA known as trans-activating RNA (tracrRNA). The duplex forms a complex with a Cas protein, such that the Cas protein acts upon the target nucleic acid sequence, for example by cleaving the selected nucleic acid sequence.

For the guide RNA to target the target nucleic acid sequence, a "protospacer adjacent motif' (PAM) region downstream of the target nucleic acid sequence is required.

Hence, the CRISPR/Cas system will be understood to refer to a guide RNA which is specific for a target nucleic acid sequence, a CAS protein and a PAM region. The CRISPR/Cas system will be understood to also comprise the further small RNA. In some embodiments the CRISPR/Cas system comprises a single guide RNA (sgRNA), the sgRNA comprising the crRNA and the tracrRNA. In some embodiments the CRISPR/Cas system further comprises a repair template. As the skilled person will appreciate, a repair template is a nucleic acid sequence, which comprises a specific nucleic acid sequence for insertion into a cleavage site.

In the context of the present invention the target nucleic acid sequence will be understood to be a nucleic acid sequence of the MYCBP2, the orthologue, or mutant, or fragment thereof. The target nucleic acid sequence may be at least 20 base pairs. In some embodiments, the target nucleic acid sequence is no more than 70 base pairs. In some embodiments, the target nucleic acid sequence is at least 20 base pairs and no more than 55 base pairs.

Advantageously, therefore, the CRISPR/Cas system may be used to modify a nucleic acid sequence of the MYCBP2, the orthologue, or mutant, or fragment thereof by cleavage and/or insertion of a specific nucleic acid sequence. The activity of the modified expressed protein or peptide can then be detected.

In embodiments wherein the test substance comprises a CRISPR/Cas system, various methods can be envisaged for contacting the MYCBP2, the orthologue, or mutant, or fragment target nucleic acid sequence. For example, contacting the MYCBP2, the orthologue, or mutant, or fragment target nucleic acid sequence may comprise contacting a cell with the CRISPR/Cas system by, for example, electroporation, transfection or transduction. The contacted MYCBP2, the orthologue, or mutant, or fragment target nucleic acid sequence can then be isolated from the cell, or provided in the form of a cell lysate.

Suitable methods for incorporating the PAM region proximal to the target nucleic acid sequence, the generation of specific guide RNA(s), tracrRNA(s) and/or sgRNA(s), and the method of using the CRISPR/Cas system will be known to the skilled person and will also be readily available from various references such as references 42-63.

Web-based tools, as detailed in references 64-66, may also be utilised to help identify suitable CRISPR target sequences

In some embodiments, the Cas protein has endonuclease activity. Thus, in such embodiments, the Cas protein is capable of cleaving the selected nucleic acid region. In other embodiments, the Cas protein is nuclease dead (i.e. has reduced or no nuclease activity). When the Cas protein is nuclease dead, the Cas protein may be attached to a transcription activator or a transcription repressor, such that the expression of the selected nucleic acid is capable of being upregulated or downregulated, respectively.

Desirably, the Cas protein comprises the Cas9 protein.

The method is suitable for testing a plurality of test substances. Advantageously, this provides a high-throughput screening assay for the fast and accurate identification of MYCBP2 modulators.

In some embodiments the hydroxyl group, of the probe may be present on an amino acid.

The interaction may comprise esterification by MYCBP2, the orthologue, or mutant, or fragment thereof of the hydroxyl group with ubiquitin.

Thus, in some embodiments, the method further comprises providing ubiquitin.

The probe may comprise a peptide. In some embodiments the probe comprises a small molecule. Exemplary proxy substrates tested herein and shown as being functional are glycerol, tris(hydroxymethyl)aminomethane, HEPES (4-(2-hydroxyethyl)-1-piperazineethanesulfonic acid), serine, threonine, and small serine/threonine containing peptides. However, any small molecule with an exposed hydroxy group maybe expected to serve as a proxy substrate.

The level of interaction between the probe and MYCBP2, the orthologue, or mutant, or fragment thereof may be determined indirectly, for example by monitoring the esterification of the probe molecule with ubiquitin.

Most known E3 ligases exhibit selectivity for lysine residues. Consequently, many E3 ligases ubiquitinate a lysine residue(s) on a substrate. Surprisingly, the present inventors have found the MYCBP2 exhibits selectivity for threonine, and, to a lesser extent, serine, rather than lysine. Hence, in some embodiments, the probe may comprise threonine or serine, preferably threonine.

The method may further comprise providing one or more other components of the ubiquitin enzymatic cascade. Other components of the ubiquitin enzymatic cascade include ATP, E1 activating enzyme, E2 conjugating enzyme (also referred to as E2 or E2 enzyme) and/or ubiquitin. By providing one or more of these components, the endogenous enzymatic cascade can be recreated in full or part. Preferably, the method further comprises providing one or more of the other components of the ubiquitin enzymatic cascade. In some embodiments the method comprises providing ATP, E1 activating enzyme, E2 and ubiquitin. The E2 enzyme may be selected from variants of UBE2D1, UBE2D2, UBE2D3, UBE2D4 and UBE2E1. In some embodiments the E2 enzyme is selected from UBE2D1, UBE2D1 C86, UBE2D1 C86 AzF3(X) and UBE2D3.

The probe may comprise an activated biological molecule corresponding to the formula (I) 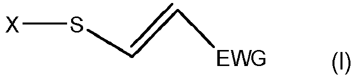 wherein X is a biological molecule and EWG is an electron withdrawing group. In this embodiment, the probe is capable of interacting with active MYCBP2 by being capable of forming a covalent bond by click-like thiol addition reaction when in proximity with a cysteine group of the active site of the active MYCBP2. In such embodiments, step c) comprises detecting formation of one or more conjugates which may be formed, wherein a modulated formation of one or more conjugates is indicative of a modulated level of interaction as compared to the level of interaction in the absence of the test substance.

Preferably a sulphur containing moiety of the biological molecule is derivatised in order to form the activated biological molecule. Typically the biological molecule may be a protein or peptide and the sulphur containing moiety is a cysteine, especially an internal cysteine present within the protein or peptide. The cysteine may be a naturally occurring cysteine in the biological molecule, or may be introduced into the molecule

The biological molecule may be a ubiquitin conjugating enzyme (E2). Preferably one or more catalytically active cysteine residues is/are derivatised. The E2 enzyme may be selected from UBE2D1, UBE2D2, UBE2D3, UBE2D4 and UBE2E1. In some embodiments the E2 enzyme is selected from UBE2D1, UBE2D1 C86, UBE2D1 C86 AzF3(X) and UBE2D3.

In embodiments wherein the biological molecule is E2, the level of interaction between the probe and MYCBP2, the orthologue, or mutant, or fragment thereof may be determined by probe- and MYCBP2-dependent discharge of ubiquitin from E2.

The electron withdrawing group (EWG) may be any suitable EWG known to the skilled addressee. Examples of suitable EWGs include -NO₂, -NR₃⁺, -CF₃, or other trihalide, - CN, -SOOR, -SOOH, -COOH, -COOR, -CHO, -COR, wherein R is typically H, NH, or C1-C4 alkyl (e.g. methyl) or alkenyl. In one example EWG is -CN, or -CO₂Me. In one example the EWG is not further substituted. In another example the EWG may be further substituted in order to, for example, provide a functional group which is capable of reacting with a moiety of another molecule and forming a covalent bond.

For example, the EWG group may be substituted with a molecule comprising an alyknyl group. Such an alkynyl group may react by a click chemistry type cycloaddtion reaction with an azide moiety to form a 1,2,3-triazole.

In one example the EWG is bound to the biological molecule Y by way of a triazole group. In accordance with this embodiment, conjugate (II), more specifically conforms to conjugate (III) below:

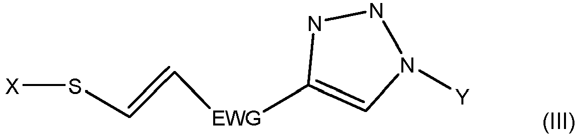

Such a conjugate may be formed in accordance with the following reaction:

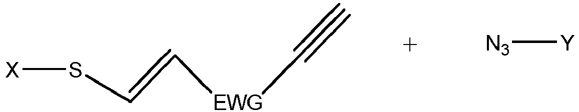

The first biological molecule X may be an enzyme and the further biological molecule Y may, for example, be a substrate or ligand for the enzyme. For example, the enzyme may be an E2 ubiquitin conjugating enzyme and the substrate is ubiquitin. In examples where the enzyme is an E2 ubiquitin conjugating enzyme and the substrate is ubiquitin, there is no requirement for the method to provide ATP, E1, unmodified E2 or the endogenous protein substrate.

The probe may be conjugated to a surface. The probe may be conjugated to the surface by passive adsorption, for example hydrophobic or hydrophobic/ionic interaction between the probe and the surface. In some embodiments the probe comprises a moiety for conjugation to the surface. For example, the probe may comprise a functional group as the moiety, which is capable of reacting with a moiety on the surface to form a covalent bond. In such embodiments the functional group of the probe may comprise an amine or sulfhydryl group. The surface moiety may comprise an amine or carboxyl group. In some embodiments the probe is conjugated to the surface by high-affinity non-covalent reactions.

The surface may comprise one or more multi-well plates, which conveniently enable high-throughput analysis. In such embodiments, active MYCBP2, the orthologue, or mutant, or fragment thereof can interact with the probe so that the active MYCBP2, the orthologue, or mutant, or fragment thereof becomes tethered to the surface. This enables detection, for example, by antibody-based detection technology such as ELISA.

The probe of the present invention may comprise a label, for example an affinity tag. The term "label" as used herein denotes a biochemical marker or tag, i.e. an easily recognizable chemical moiety, e.g. a protein, peptide, or small molecule. The label may be covalently attached to the probe. In embodiments wherein the probe comprises an N and a C terminus, for example where the probe comprises a peptide, the label may be covalently attached to the N or C terminus, preferably the N-terminus. Numerous labels are known to the skilled addressee and include affinity labels, e.g. affinity tags (Kimple and Sondek, BioTechniques (2002), 33:578-590), fluorophores (such as TAMRA, DAPI, fluorescein, Cy3, Cy5, SYBR green and the like), biotin, epitope tags or radioactive labels.

In some embodiments, the method is carried out in the absence of endogenous MYCBP2 protein substrate. The method may be carried out in the absence of one or more other components of the ubiquitin enzymatic cascade, for example one or more of ATP, E1 and/or E2 ligases. In some embodiments, the method does not comprise providing an endogenous MYCBP2 substrate, ATP, E1 and/or E2. Advantageously, this provides a method requiring only minimal reagents that can be carried out with minimum cost and complexity.

The method may further comprise the preliminary step of providing the MYCBP2, the orthologue, or mutant, or fragment thereof. In some embodiments, the MYCBP2, the orthologue, or mutant, or fragment thereof is endogenous, for example provided in the form of a cell lysate. This provides physiological context to the method. For example, in embodiments where step a) comprises contacting a mutant of MYCBP2 with a test substance, the mutant may have been isolated from a particular subject. The method can therefore advantageously be used to identify MYCBP2 modulators specific for the particular subject. Where the modulator(s) can be used in the therapy and/or prophylaxis of the subject, this provides a method of obtaining personalised therapy for the subject.

In other embodiments, the MYCBP2, the orthologue, or mutant, or fragment thereof may be recombinant. By "recombinant" it will be understood that this refers to MYCBP2, the orthologue, or mutant, or fragment thereof which has been genetically engineered, for example comprising or being expressed from a nucleic acid construct.

The skilled person will therefore appreciate that the term "recombinant" defines the endogenous MYCBP2, the orthologue, or mutant, or fragment thereof provided and/or expressed in a nucleic acid construct, and/or a modified MYCBP2, the orthologue, or mutant, or fragment thereof provided and/or expressed in a nucleic acid construct.

In some embodiments, the MYCBP2, the orthologue, or mutant, or fragment thereof may be provided in overexpressed form in the method. Overexpressed will be understood to refer to increased expression levels relative to the endogenous expression levels. Typically, overexpression is achieved by expressing a nucleic acid construct comprising the MYCBP2, orthologue, mutant or fragment gene or cDNA thereof.

In some embodiments step a) comprises contacting a fragment of MYCBP2 comprising at least the active site of the MYCBP2 native protein with a test substance. By fragment, we include that the fragment of MYCBP2 can vary from the naturally occurring nucleotide or peptide sequence with the proviso that the fragment substantially retains the biological activity of MYCBP2. By retain the biological activity of MYCBP2 it is meant that the fragment retains at least a portion of the enzymatic activity as compared to the native MYCBP2. Typically the fragment retains at least 50%, such as 60%, 70%, 80% or 90% activity. In some instances the fragment may have a greater enzymatic activity than the native MYCBP2. In some embodiments the fragment may display an increase in another physiological feature as compared to the native enzyme. For example, the fragment may possess a greater half-life *in vitro* and/or *in vivo,* as compared to the native enzyme.

Without wishing to be bound by theory, the inventors believe that the residues C4520 and C4572 of the full-length native MYCBP2 protein are required for the transthiolation between E2-ubiquitin and MYCBP2, and the subsequent relay of ubiquitin. Hence, it will be understood that the fragment comprises at least the active site of the MYCBP2 active protein. The fragment comprises at least the residues C4520 to C4572 of the full-length native MYCBP2 protein. Without wishing to be bound be theory the inventors believe that the new teaching and unique nature of the cooperative mechanism adopted by C4520 and C4572, and the reactivity of the cysteine residues, makes these sites privileged features for targeting with modulators that may have therapeutic benefit.

In addition to the active site, it is preferred if the fragment has a sequence which has at least 5%, at least 10%, at least 20%, at least 30%, at least 40%, at least 50%, at least 60%, at least 70%, at least 80% or at least 90% of the native sequence.

In some embodiments, the fragment further comprises the RING domain of MYCBP2, which is understood to be residues 4390-4441 of the full-length native MYCBP2 protein.

In some embodiments, the fragment's C-terminus is truncated relative to the full-length native MYCBP2 protein.

In some embodiments, the fragment comprises or consists of residues 4390 to 4572 of the full-length native MYCBP2 protein.

Preferably, the fragment's N-terminus comprises or consists of residue 4390. In other embodiments, the fragment's N terminus comprises or consists of residue 4378.

In some embodiments, the fragment comprises or consists of residues 4378 to 4572.

In some embodiments, the fragment's N terminus comprises residue 3224 of the full-length native MYCBP2 protein. In other embodiments, the fragment's N terminus comprises residue 3156 of the full-length native MYCBP2 protein.

In some embodiments, the fragment's C-terminus comprises any residue of from 4572 to 4640 of the full-length native MYCBP2 protein.

In some embodiments, the fragment comprises or consists of residues 4390 to 4640 of the full-length native MYCPB2 protein.

In some embodiments, the fragment comprises or consists of residues 4378-4640 of the full-length MYCBP2 protein. This fragment comprises the RING domain, the active site and the subsequent C-terminal residues. The inventors have found that this fragment will efficiently esterify the hydroxyl probes of the present invention, for example a probe comprising a hydroxyl group, with ubiquitin.

In some embodiments, the fragment comprises or consists of isoform 2 of MYCBP2. This will be understood to refer to the full-length canonical isoform of MYCBP2 minus residues 3901 to 3903.

The fragment may comprise a fragment of an orthologue.

Various orthologues of MYCBP2 will be known to the skilled person. For example, orthologues of MYCBP2 may include, but not be limited to mouse MYCBP2, zebrafish Esrom/phr1, Drosophila Highwire and C.elegans RPM-1.

In some embodiments step a) comprises contacting an orthologue of MYCBP2 with a test substance. The orthologue may be selected from mouse Phr1, zebrafish Esrom/phr1, Drosophila Highwire and C.elegans RPM-1. In some embodiments the orthologue comprises or consists of Drosophila Highwire.

Preferably, the method is an *in vitro* method.

Detection of the level of interaction of the probe with MYCBP2, the orthologue, or mutant, or fragment thereof may be any suitable analytical technique, various techniques being known to the skilled person.

Detection may be using magnetic separation, immunological separation, gel filtration chromatography, affinity chromatography, column chromatography, displacement chromatography, electro chromatography, gas chromatography, high performance liquid chromatography, ion chromatography, micellar electrokinetic chromatography, normal phase chromatography, paper chromatography, reversed-phase chromatography, size exclusion chromatography, thin layer chromatography, gel electrophoresis, centrifugation, adhesion, flow cytometry, or other techniques known to the skilled addressee.

The detection may be carried out, for example by reducing SDS gel electrophoresis and immunoblotting with an antibody specific for the probe or tag bound thereto. Alternatively detection may be carried out by performing mass spectrometry.

In embodiments where the interaction comprises esterification of the hydroxyl group of the probe with ubiquitin (by MYCBP2, the orthologue, or mutant, or fragment thereof), any suitable techniques that allow the resolution of unmodified ubiquitin from the ubiquitin adducted probe may be used. For example, the hydroxyl group may be labelled with an appropriate reporter and the reporter detected. The reporter may include, but may not be limited to, a fluorophore, epitope tag or biotin.

Alternatively, a "catch and release" detection technique may be suitable in embodiments where the interaction comprises esterification by MYCBP2, the orthologue, or mutant, or fragment thereof of the hydroxyl group of the probe with ubiquitin. Without wishing to be bound by theory, the inventors believe that following interaction, the ester linkage between ubiquitin and the hydroxyl group is labile. A catch and release detection technique could employ this lability by facilitating ester cleavage. For example, ester cleavage could be mediated by a base such as hydroxide or hydroxylamine. The cleaved ubiquitin and/or hydroxyl probe may be detected by any suitable analytical technique, for example by ELISA or fluorescence methods such as FRET or fluorescence polarization.

In embodiments where the probe comprises an activated biological molecule, detection may be by any suitable technique, which can resolve the unmodified activated biological molecule from activated biological molecule bound to the MYCBP2, the orthologue, or mutant, or fragment thereof. For example, the activated biological molecule may comprise a label, which can be detected by fluorescence polarisation. In embodiments wherein the activated biological molecule comprises an E2-ubiquitin conjugate probe, detection may be by resolving the discharged ubiquitin from E2 using any suitable analytical technique.

Other forms of detection could include that the activated biological molecule and the MYCBP2, the orthologue, or mutant, or fragment thereof may each comprise a label. Once both labels are in close proximity, binding of the activated molecule to the MYCBP2, the orthologue, or mutant, or fragment thereof may be detected by FRET (Fluorescence Resonance Energy Transfer) AlphaScreen (Perkin Elmer) or HTRF (Homogenous Time-Resolved Fluorescence) technology.

An MYCBP2 modulator as identified in accordance with the invention may find use in a method of treating or preventing neuronal damage, wherein the MYCPB2 modulator is capable of interacting with the active site of MYCBP2.

As described above, the inventors believe that the residues C4520 and C4572 of the full-length native MYCBP2 protein are required for the transthiolation between E2-ubiquitin and MYCBP2, and the subsequent relay of ubiquitin. Hence, the active site will be understood to refer to include residues C4520 and C4572.

The inventors have observed that MYCBP2 can ubiquitinate the non-lysine serine, and preferably threonine residues of endogenous substrates. For example, the inventors have found that MYCBP2 can ubiquitinate Nicotinamide Mononucleotide Adenyltransferase (NMNAT2), an enzyme implicated in axonal degeneration, by esterification. The inventors' observations reveal potential new roles for MYCBP2 in neuronal damage. Accordingly, the present invention contemplates the use of modulators of MYCBP2, which are capable of modulating MYCBP2 activity, for example, as may be identified from the method of identifying modulators described above.

Without wishing to be bound by any particular theory, neuronal damage such as axonal degeneration may be due at least in part to the ability of MYCBP2 to ubiquitinate neuronal-associated molecules such as NMNAT2. Desirably, therefore, the modulator may be an inhibitor of MYCBP2.

Hence, in some teachings the modulator decreases the activity of MYCBP2. The activity of MYCBP2 may be reduced by the modulator by at least 10%, at least 20%, at least 30%, at least 40%, at least 50%, at least 60%, at least 70%, at least 80%, at least 90% or 100%.

As used herein "treating" or "treatment" refers to reducing or alleviating symptoms associated with the neuronal damage. As used herein "preventing" or "prevention" refers to protecting a subject from neuronal damage.

The modulator may be capable of interacting with the residue C4520 and/or C4572 of MYCBP2, for example by binding. Preferably, by interacting with one or more of these residues, the active site of MYCBP2 is blocked and so MYCBP2 activity is reduced or prevented.

The neuronal damage may be from trauma or as a side-effect of a neurotoxic therapy, for example chemotherapy. As used herein, "trauma" will be used to define neuronal injury, for example as a result of a physical force to the subject, such as a fall or being struck with an object. Neuronal damage may also be acquired such as from side effects from clinically used neurotoxic medicines such as those employed in cancer chemotherapy

In some embodiments the neuronal damage is as an effect of a disorder such as a neurological disorder, diabetes, HIV (Human immunodeficiency virus) infection, AIDS (acquired immunodeficiency syndrome) and/or ischemia.

In some embodiments the neuronal damage is from one or more neurological disorder(s). "Neurodegenerative disorder" will be understood to refer to disorder in which neurons degenerate in function and/or structure and/or die. Degeneration is typically gradual but in some instances may be sudden. Example neurodegenerative diseases include, but are not limited to, Alzheimer's disease, Diffuse Lewy Body disease, Fronto-temporal dementia (FTD) (Pick's disease) (including FTD subtypes behavioural variant/frontal variant Fronto-temporal dementia, semantic dementia and progressive non-fluent aphasia), Corticobasal degeneration, Argyrophilic Grain disease, Parkinson's disease, Parkinson's disease dementia, Perry syndrome, Familial British dementia, Familial Danish dementia, Progressive Supranuclear Palsy, Multiple System Atrophy, Lewy Body disease (Dementia with Lewy bodies), Huntington's disease, Spinobulbar Muscular Atrophy (Kennedy's disease), Dentatorubral-pallidoluysian Atrophy, Spinocerebellar ataxias 1,2,3,6,7 and 17, Motor Neurone disease (Amyotrophic Lateral Sclerosis), Multiple Sclerosis (MS), Prion diseases including Creutzfeldt-Jakob disease, variant Creutzfeldt-Jakob disease (Bovine spongiform encephalopathy), Kuru, Fatal Familial Insomnia, Gertsmann-Straussler-Scheinker syndrome, Charcot-Marie Tooth diseases, optic neuropathies such as Glaucoma, and Variable Protease Sensitive prionopathy. Other neurodegenerative diseases will be known to a skilled person.

Human symptoms of neurodegenerative disorder may include dementia (including memory loss and/or a reduction in cognitive capacity as measured by standard tests known to a skilled person) mood changes, reduced mobility, reduced or no speech quality, clumsiness, difficulty balancing, uncontrolled movements, trembling limbs, stiff limbs or decreased speed of movement. In the context of human symptoms, "reduced" refers to a decreased value, relative to individuals without the neurodegenerative disease. Other human symptoms will be known to a skilled person.

The neurodegenerative disorder may be selected from one or more of Alzheimer's disease, Diffuse Lewy Body disease, Amyotrophic Lateral Sclerosis (ALS), Fronto-temporal dementia (FTD) (Pick's disease) (including FTD subtypes behavioural variant/frontal variant Fronto-temporal dementia, semantic dementia and progressive non-fluent aphasia), Corticobasal degeneration, Argyrophilic Grain disease,, Parkinson's disease, Parkinson's disease dementia, Perry syndrome, Familial British dementia, Familial Danish dementia, Progressive Supranuclear Palsy, Multiple System Atrophy, Lewy Body disease (Dementia with Lewy bodies), Huntington's disease, Spinobulbar Muscular Atrophy (Kennedy's disease), Dentatorubral-pallidoluysian Atrophy, Spinocerebellar ataxias 1,2,3,6,7 and 17, Motor Neurone disease (Amyotrophic Lateral Sclerosis), Multiple Sclerosis (MS), Prion diseases including Creutzfeldt-Jakob disease, variant Creutzfeldt-Jakob disease (Bovine spongiform encephalopathy), Kuru, Fatal Familial Insomnia, Gertsmann-Straussler-Scheinker syndrome, Charcot-Marie Tooth diseases, optic neuropathies such as Glaucoma, and Variable Protease Sensitive prionopathy.

In some embodiments the neurodegenerative disorder is selected from one or more of Alzheimer's disease, Parkinson's disease, Amyotrophic Lateral Sclerosis (ALS) Huntington's disease, Motor Neurone disease, Gertsmann-Straussler-Scheinker syndrome, Charcot-Marie Tooth diseases, optic neuropathies such as Glaucoma, Creutzfeldt-Jakob (prion) disease and Multiple Sclerosis (MS).

In some embodiments, the neurodegenerative disorder is selected from one or more of Alzheimer's disease, Parkinson's disease, Amyotrophic Lateral Sclerosis (ALS) Huntington's disease, Motor Neurone disease, Creutzfeldt-Jakob (prion) disease and Multiple Sclerosis (MS).

The neurodegenerative disorder may be selected from one or more of Alzheimer's disease, Parkinson's disease, Amyotrophic Lateral Sclerosis (ALS) Huntington's disease and Motor Neurone disease.

In some embodiments the neurodegenerative disorder is selected from one or more of Alzheimer's disease, Parkinson's disease and Huntington's disease.

The neurodegenerative disorder may be selected from one or more of Alzheimer's disease, Parkinson's disease, Amyotrophic Lateral Sclerosis (ALS) and Huntington's disease. In some embodiments, the neurodegenerative disorder is selected from Alzheimer's disease or Parkinson's disease.

In some embodiments, the modulator comprises a peptide, a protein, an enzyme, an aptamer, an antibody or an antibody fragment (such as a Fab or F(ab')2) fragment, an scFV antibody or any other functional antigen-binding fragment.

In some embodiments the modulator comprises or consists of an antibody, an antibody fragment, a peptide or an aptamer.

The modulator may be an antibody or an antibody fragment

In embodiments wherein the modulator comprises an aptamer, the aptamer may comprise DNA or RNA.

MYCBP2 modulators identified in accordance with the present invention may find use in a method of treating or preventing neuronal damage, wherein the MYCPB2 modulator is capable of disrupting the binding of MYCBP2 to its natural substrate or ligand

Disrupting a nucleic acid sequence may comprise cleavage of the nucleic acid sequence, deletion of a portion of the nucleic acid sequence, degradation of the nucleic acid sequence, destabilization of the nucleic acid sequence and/or insertion of a further nucleic acid sequence into the nucleic acid sequence.

In some embodiments the modulator comprises an siRNA, an miRNA or a CRISPR/Cas system. In some embodiments, the modulator comprises a CRISPR/Cas9 system.

In embodiments where the modulator comprises an siRNA, an miRNA or a CRISPR/Cas system as described herein, the method of treating or preventing neuronal damage may comprise isolating a cell(s) from a subject, contacting the cell(s) with the siRNA, miRNA or CRISPR/Cas system and administering the contacted cell(s) to the subject.

In embodiments wherein the modulator is a peptide or protein, a nucleic acid sequence encoding the peptide or protein may be provided in a suitable vector, for example a plasmid, a cosmid or a viral vector. The vector (i.e. a construct), comprises a nucleic acid sequence which encodes the protein or peptide. The nucleic acid sequence is preferably operably linked to a suitable promoter.

Modulators which are nucleic acids, such as siRNAs, miRNAs or the CRISPR/Cas system, may be modified (e.g. via chemical modification of the nucleic acid backbone), or delivered in suitable delivery system which protects the nucleic acids from degradation and/or immune system recognition. Examples of suitable delivery systems include nanoparticles, lipid particles, polymer-mediated delivery systems, lipid-based nanovectors and exosomes.

Embodiments of the invention, together with examples for background or reference purposes will now be described by way of example and with reference to the accompanying figures, in which:
Figure 1 shows the activity-based proteomics of E3 ligases;
Figure 2 shows the LC-MS characterization of biotinylated ABP intermediates and biotinylated ABPs;
Figure 3 shows the activity-based proteomic profiling of neuroblastoma SH-SY5Y cells;
Figure 4 shows MYCBP2 is a novel class of E3 ligase and data support of a cysteine relay mechanism;
Figure 5 shows ABPs label C4520 within MYCBP2_{cat} with high selectivity;
Figure 6 shows the esterification activity of MYCBP2_{cat} and further data in support of a dual cysteine mechanism that operates in *cis*;
Figure 7 shows that MYCBP2 ubiquitinates serine and threonine with selectivity for threonine;
Figure 8 shows that MYCBP2 has serine/threonine Ub esterification activity but has preference for threonine;
Figure 9 shows the E2 requirements of MYCBP2;
Figure 10 is a structural comparison and representative stereo views of the MYCBP2_{cat} crystallographic model;
Figure 11 shows the crystal structure of MYCBP2_{cat};
Figure 12 shows the structural basis for threonine selectivity, model of an E2-E3 intermediate and model of Ub relay;
Figure 13 shows the modelling of E2-MYCBP2_{cat} complex; and
Figure 14 is a schematic representation for proposed model for RCR E3 ligase mechanism.

### Detailed description

### Introduction

Ubiquitination is initiated by ubiquitin (Ub) transfer from an E1 activating enzyme (E1) to an E2 conjugating enzyme (E2) producing a covalently linked intermediate (E2~Ub)¹. E3 ligases (E3s) of the Really Interesting New Gene (RING) class recruit E2~Ub via their RING domain and mediate direct transfer of Ub to substrates². Homologous to E6-AP Carboxy Terminus (HECT) E3s undergo a catalytic cysteine-dependent transthiolation reaction with E2~Ub forming a covalent E3~Ub intermediate^{3,4}. Additionally, RING-between-RING (RBR) E3s have a canonical RING domain that is linked to an ancillary domain. This contains a catalytic cysteine enabling a hybrid RING/HECT mechanism⁵. Ubiquitination is typically considered a posttranslational modification of lysine residues as human E3s endowed with non-lysine activity remain to be discovered. Herein, we carry out activity-based protein profiling of HECT/RBRlike E3s and uncover the neuron-associated E3 MYCBP2/Phr1 as a novel class of RING-linked E3 with esterification activity and intrinsic selectivity for threonine over serine. MYCBP2 contains two essential catalytic cysteine residues which relay Ub to substrate via thioester intermediates. Crystallographic characterization of this new class of E3 ligase, which we designate as an RING-Cys-Relay (RCR), reveals insights into its mechanism and threonine selectivity. These findings implicate cellular regulation of higher eukaryotes by non-lysine ubiquitination and unappreciated mechanistic diversity of E3 enzymes.

### Materials and Methods

### General materials

All DNA constructs were verified by DNA sequencing, (Medical Research Council Protein Phosphorylation and Ubiquitylation Unit, University of Dundee). DNA for bacterial protein expression was transformed into *E. coli* BL21-DE3 (Merck). All cDNA plasmids and antibodies generated for this study are available to request through our reagents website (https://mrcppureagents.dundee.ac.uk/). All solvents and reagents were purchased from Sigma-Aldrich or VWR unless otherwise stated.

### Biotin functionalized ABP preparation

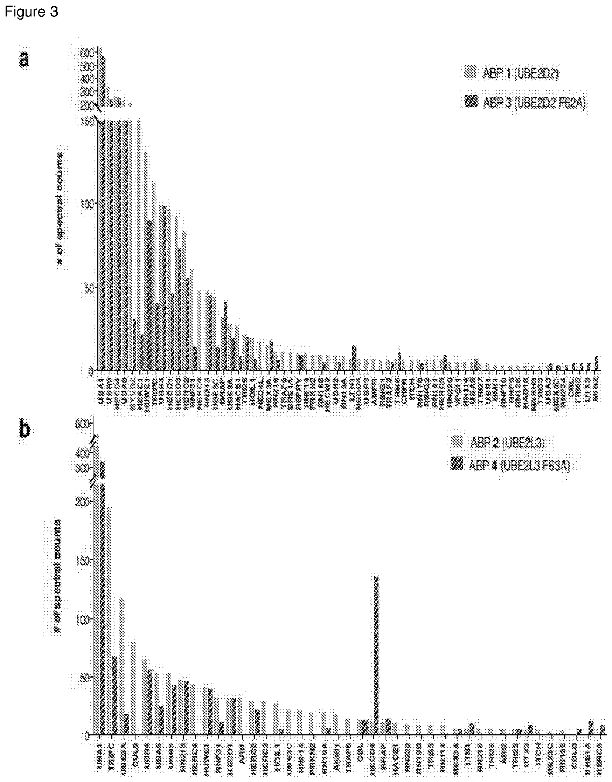

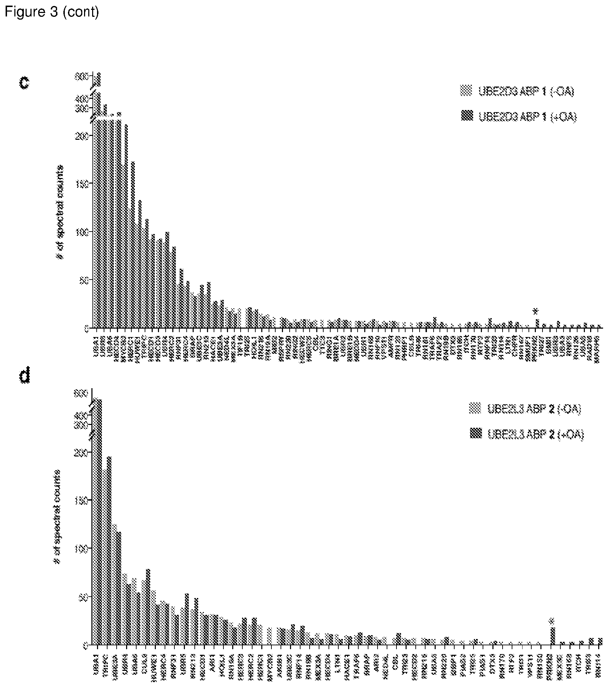

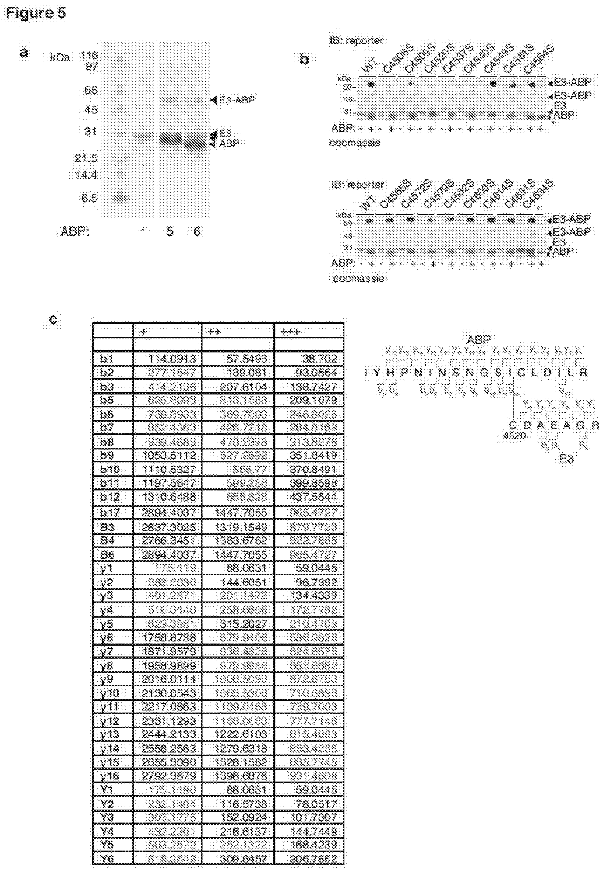

Ub with a GCSSG N-terminal extension was expressed from plasmid *pTXB1-UbΔ74-*76-T3C plasmid⁷. An equivalent plasmid encoding Ub residues 1-74 (*pTXB1-UbΔ75-*76-T3C) was also created. Ub thioesters were obtained as described previously generating cysteine tagged Cys-Ub₁₋₇₃-SR and Cys-Ub₁₋₇₄-SR, respectively⁷. The extended Ub₁₋₇₄ was included as this retains Arg74 which forms a favourable electrostatic interaction with the RBR E3 HOIP³¹. Cys-Ub₁₋₇₃-SR (30 mg) was reconstituted by the addition of DMSO (116 µL) followed by H₂O (456 µL). An aqueous stock solution (48 mM) of EZ-link lodoacetyl-PEG2-biotin (Thermofisher) was prepared and 200 µL was added to the Cys-Ub₁₋₇₃-SR solution (580 µl) followed by the addition of 900 µl degased buffer (50 mM Na₂HPO₄ pH 7.5, 150 mM NaCl). The reaction was incubated at 23 °C for 1 hour and monitored by LC-MS. The protein (Biotin-Ub₁₋₇₃-SR) was then further purified by semi-preparative RP-HPLC (Column: BioBasic-4; Part number: 72305-259270). A gradient of 20 % buffer A to 50 % buffer B was applied at a flow rate of 10 mL min⁻¹ over 60 min (buffer A=0.1 % TFA in H₂O, buffer B=0.1 % TFA in acetonitrile). The above procedure was repeated to generate Biotin-Ub₁₋₇₄-SR. HPLC fractions containing Biotin-Ub_{1-7X}-SR were pooled and lyophilized (Yield: Biotin-Ub₁₋₇₃-SR 75-85 %, Biotin-Ub₁₋₇₄-SR 40-50 %) **(****Figure 3a** **and d).** Biotin-tagged ABPs containing thioacrylamide warheads were then prepared as previously described⁷ employing the E2 recognition elements UBE2D2*, UBE2D2* F62A, UBE2L3* and UBE2L3* F63A furnishing ABPs **1, 3, 2** and **4 (****Figure 3b**, **c****, e and f**). *denotes E2 where non-catalytic Cys residues were mutated to Ser. ABPs based on UBE2D2* and UBE2D3* bearing hexahistidine reporter tags and thioacrylamide warheads **(****Figure 5a****),** were also prepared yielding ABPs **5** and **6,** respectively.

### Cell culture and lysis Protocol

SH-SY5Y cells were cultures as previously described⁷. HEK293 were cultured (37 °C, 5 % CO₂) in Dulbecco's Modified Eagle Medium (DMEM) supplemented with 10 % (v/v) Fetal Bovine Serum (FBS), 2.0 mM L-glutamine, and antibiotics (100 units mL⁻¹ penicillin, 0.1 mg mL⁻¹ streptomycin). Cell transfections were performed using polyethylenimine (Polysciences) according to the manufacturer's instruction. MG-132 (50 µM) was added to cells two hours prior to lysis. Cells were rinsed with ice-cold PBS and extracted in lysis buffer (1 % NP-40, 50 mM Tris-HCI pH 7.5, 1.0 mM EGTA, 1.0 mM EDTA, 0.27 M sucrose, 10 mM sodium 2-glycerophosphate, 0.2 mM phenylmethane sulfonyl fluoride (PMSF), 1.0 mM benzamidine, 1.0 mM sodium orthovanadate, 50 mM sodium fluoride and 5.0 mM sodium pyrophosphate, 50 mM iodoacetamide and cOmplete^{™}, EDTA-free protease inhibitor cocktail (Roche)). Lysates were then clarified by centrifugation at 4 °C for 30 min at 14,800 rpm. Supernatants were collected (total cell extracts) and protein concentration determined by Bradford assay. For the base-lability test, indicated cell lysates were further incubated with 0.5M hydroxylamine, pH 9.0 at 37 °C for 30 minutes.

### Immunoblotting

Samples were mixed with NuPAGE LDS sample buffer (Thermofisher) without boiling, and resolved by SDS-PAGE (4-12 % NuPage gel, Thermofisher) with MOPS or MES running buffer and transferred on to 0.45 µm nitrocellulose membranes (GE Life Sciences). Membranes were blocked with PBS-T buffer (PBS + 0.1 % Tween-20) containing 5 % (w/v) non-fat dried skimmed milk powder (PBS-TM) at room temperature for 1 h. Membranes were subsequently probed with the indicated antibodies in PBS-T containing 5 % (w/v) Bovine Serum Albumin (BSA) overnight at 4 °C. Detection was performed using HRP-conjugated secondary antibodies in PBS-TM for 1 h at 23 °C. ECL western blotting detecting reagent (GE Life Sciences) was used for visualization in accordance with the manufacturers protocol.

### Antibodies

His-tagged species were probed with 1:10000 anti-His primary antibody (Clontech, #631212). Alpha tubulin (1E4C11) mouse mAb (Proteintech^{®}) was used at 1:10000 dilution. The MYCBP2 antibody was used at 0.5 µg mL⁻¹ and raised by in sheep by MRC PPU Reagents and Services and affinity-purified against the indicated antigen: anti-MYCBP2 (2nd bleed of SA357, residues 4378-4640 of human MYCBP2). Mouse monoclonal NMNAT2 antibody (clone 2E4; Sigma Aldrich) was used at 0.5 µg mL⁻¹.

### Activity-based proteomic profiling of SH-SY5Y cells

SH-SY5Y total cell lysate (4.5 mg, 550 µL) was mixed with ABPs **1, 2, 3** and **4** (3 µM) and incubated at 30 °C for 4 hours. To induce Parkin activation cells were administered with oligomycin (5 µM) and antimycin A (10 µM) (OA) for 3 hours. Control enrichments were also performed where probe was withheld. Extracts were mixed with 100 µL of Pierce^{™} Streptavidin Plus UltraLink^{™} Resin (ThermoFisher Scientific) and diluted with 6 % SDS solution (20 µL) to a final concentration of 0.2 % in phosphate buffer. Samples were incubated for 4 hours at 4 °C and resin washed (2 ml 0.2 % SDS/PBS, 2 ml PBS, 1 ml 4 M urea/PBS, 2 ml PBS) and then resuspended in 190 µl Tris buffer (50 mM Tris pH 8, 1.5 M urea). Resin-bound proteins were reduced with TCEP (5 mM) for 30 minutes at 37 °C and then alkylated with iodoacetamide (10 mM) at 23 °C for 20 minutes. DTT (10 mM) was then added followed by washing with buffer (50 mM Tris pH 8, 1.5 M urea) to a final volume of 300 µL. Trypsin (2 µg) was then added and further incubated at 37 °C for 14 hours. Trifluoroacetic acid was added to a final concentration of 0.1 % and samples were desalted with a C¹⁸ MacroSpin column (The Nest Group Inc). LC-MS/MS analysis was performed on an LTQ Orbitrap Velos instrument (Thermo Scientific) coupled to an Ultimate nanoflow HPLC system (Dionex). A gradient running from 3 % solvent B to 99 % solvent B over 345 min was applied (solvent A = 0.1 % formic acid and 3 % DMSO in H₂O; solvent B = 0.08 % formic acid and 3 % DMSO in 80 % MeCN).

### Data processing

Raw files were searched against the Swissprot database and a decoy database using the MASCOT server (Matrix Science). Trypsin specificity with up to three missed cleavages was applied. Cysteine carbamylation was set as a fixed modification and variable modifications were methione oxidation/dioxidation. A PERL script was used to extract the number of rank 1 peptides for each protein from the MASCOT search results and this figure was used as the number of spectral counts. A second PERL script filtered the data by searching the human swisspfam_v30 database using the E3 domain terms RING, HECT, IBR and zf-UBR. Manual curation was also carried out which involved the addition of E1 enzymes. Any proteins with less than 3 spectral counts and less than 14-fold spectral count enrichment, relative to control experiments where ABP was withheld, were omitted from the list. Pairwise datasets were then plotted as column charts in Prism (Graphpad Software).

### Cloning of MYCBP2cat

Human MYCBP2 (NM_015057.4) sequences were amplified from full-length Addgene plasmid #2570. Wild type and mutant fragments were subcloned as BamHI/Not1 inserts into pGEX6P-1 (GE Life Sciences) for bacterial expression, or a modified version of pcDNA TM5/FRT/TO (ThermoFisher) containing an N-terminal Myc tag for mammalian expression.

### UBE1 and E2 expression and purification

6His-UBE1 was expressed in Sf21 cells and purified via its tag as previously described³². Phosphate Buffered Saline was used throughout the purification and hydroxy containing compounds avoided. UBE2D3 was expressed as an N-terminally 6His-tagged protein in BL21 cells and purified over Ni-NTA-agarose and finally dialysed into 50 mM Na₂HPO₄ pH 7.5, 150 mM NaCl, 0.5 mM TCEP. UBE2A was expressed as a GST-fusion in *E. coli* and the GST tag was proteolytically removed. The remaining E2s were expressed as recombinant bacterial proteins and purified via their His-tags and buffer exchanged by size exclusion chromatography into running buffer (50 mM Na₂HPO₄ pH 7.5, 150 mM NaCl, 0.5 mM TCEP, 0.015 % Brij-35) using a Superdex 75 column (GE Life Sciences).

### Expression and purification of MYCBP2 and GST-MYCBP2

GST-tagged MYCBP2_{cat} (Ser4378-Phe4640), wt and mutants, were expressed at 16 °C overnight and purified against glutathione resin (Expedeon) using standard procedures. GST-tagged constructs were eluted with glutathione and untagged constructs were obtained by on-resin cleavage with Rhinovirus 3C protease. Proteins were buffer exchanged into 50 mM Tris-HCI pH 7.5, 150 mM NaCl, 1.0 mM TCEP buffer and flash frozen for storage at -80 °C.

### Expression and Purification of NMNAT2

NMNAT2 was expressed with a 6His-SUMO tag in BL21(DE3) cells, induced with 0.1 mM IPTG and incubated for expression at 16 °C. The cells were collected and lysed in buffer (50mM Tris-HCI (pH 7.5), 250 mM NaCl, 0.2 mM EGTA, 20 mM imidazole, 20 mM L-arginine, 0.015% Brij 35, 1 mM Leupeptin, 1 mM Pefabloc, 1 mM DTT using standard protocols and the protein was purified over Ni-NTA-agarose. The eluted protein was incubated with His-SENP1 protease during dialysis against PBS, 20 mM L-arginine, 1 mM DTT. The tag and protease were depeleted against Ni-NTA-agarose and NMNAT2 was concentrated and subjected to chromatography on a Superdex 75 HR 10/30 into buffer (PBS, 20 mM L-arginine).

### Activity-based protein profiling of MYCBP2 cysteine mutants

The indicated MYCBP2 mutant was diluted into Tris buffer (50 mM Tris-HCI pH 7.5, 150 mM NaCI) to a final concentration of 3 µM. Probe 6 was added (12 µM) and incubated with E3 ligase at 30 °C for four hours. Reactions were quenched by the addition of 4X LDS loading buffer (supplemented with -680 mM 2-mercaptoethanol) and samples were resolved by SDS-PAGE (4-12 % NuPage gel) followed by Coomassie staining or anti-His immunoblotting.

### Tryptic MS/MS sequencing of probe-labelled MYCBP2

Crosslinking MS using ABP 6 was carried out as previously described⁷. In summary Coomassie stained SDS-PAGE band corresponding to ABP-labelled WT MYCBP2 was analyzed by LC-MS/MS using an Orbitrap Fusion^{™} Tribrid^{™} mass spectrometer (Thermo Scientific) coupled to an Ultimate nanoflow HPLC system (Dionex). A gradient running from 0 % solvent A to 60 % solvent B over 120 min was applied (solvent A = 0.1 % formic acid in H₂O; solvent B = 0.08 % formic acid in 80 % MeCN). Fragment ions were generated by HCD and 1⁺, 2⁺ and 3⁺ precursor ions excluded. Raw data was searched using the pLink software³³ against UBE2D3* and MYCBP2 sequences with trypsin specificity (up to 2 missed cleavages). The error window for MS/MS fragment ion mass values was set to the software default of 20 ppm. A crosslinker monoisotopic mass of 306.1805 Da was manually added which accounted for the theoretical mass difference associated with formation of a bisthioether between 2 Cys residues derived from probe 6, which was based on UBE2D3* and contained a thioacrylamide AVS warhead⁷.

### Tris/glycerol-mediated E2 discharge assay

Assays were carried out in buffer (50 mM Tris-HCI pH 7.5, 150 mM NaCl, 0.5 mM TCEP, 5 mM MgCl₂) containing the indicated MYCBP2 mutant (15 µM), UBE1 (1.5 µM), UBE2D3 (15 µM), Ub (37 µM) and ATP (10 mM). The reactions were incubated at 37 °C for 30 minutes. Reactions were terminated by the addition of 4X LDS loading buffer (with and without -680 mM 2-mercaptoethanol). A C4572S sample was further incubated with 0.14 N NaOH at 37 °C for 20 minutes and samples were resolved by SDS-PAGE (4-12 % NuPage gel) and visualized by Coomassie staining.

### LC-MS analysis of nucleophile discharge assays

Reactions were prepared as described for discharge assay. After 30 minutes, the reaction was analyzed using an Agilent 1200/6130 LC-MS system (Agilent Technologies) using a 10-75 % gradient over 20 minutes (buffer A = H₂O + 0.05 % TFA, buffer B = acetonitrile + 0.04 % TFA).

### Preparation of Cy3b-Ub

Ub bearing a TEV protease-cleavable N-terminal hexahistidine tag followed by a ACG motif was expressed in bacteria from a pET plasmid (kindly provide Ronald Hay, University of Dundee). Protein was purified by Ni affinity chromatography, cleaved from the tag with TEV protease then buffer exchanged into reaction buffer (50 mM HEPES, pH 7.5, 0.5 mM TCEP). Protein was concentrated to 2 mg mL⁻¹ and 221 µL (50 nmol) was mixed with Cy3b-maleimide (150 nmol, GE Life Sciences) in a final volume of 300 µL and agitated for 2 h at 25 °C. Labelled protein was then further purified with a P2 Centri-Pure desalting column (EMP Biotech) with degassed buffer (50 mM Na₂HPO₄, 150 mM NaCl).

### MYCBP2 thioester/ester trapping assay

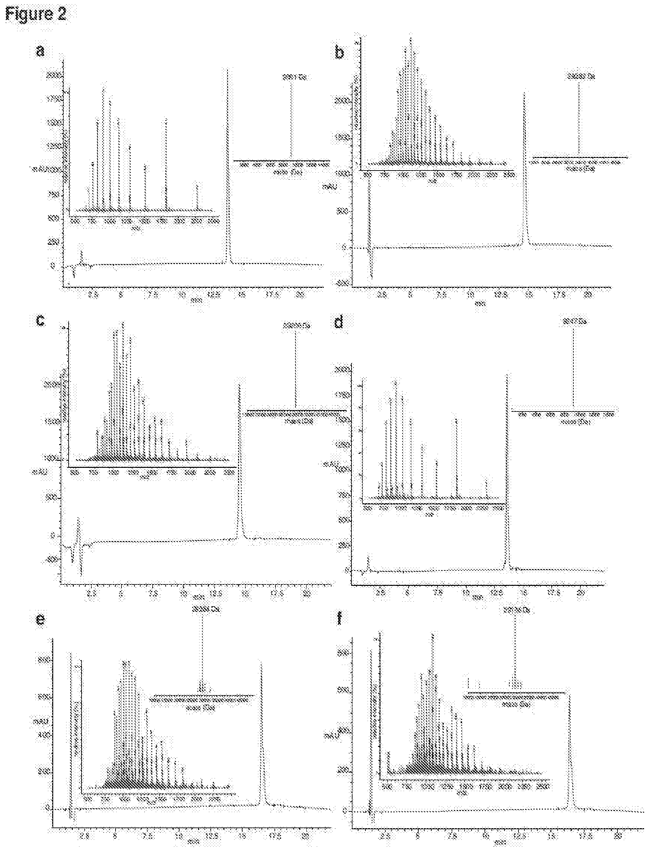

UBE1 (2 µM) was mixed with Cy3b-Ub (1 µM) in buffer (40 mM Na₂HPO₄-HCl pH 7.5, 150 mM NaCl, 0.5 mM TCEP, 5 mM MgCl₂) (Figure 2e, *Lane* 1, 2). The reaction was then initiated by the addition of ATP (5 mM) and incubated for 10 minutes at 25°C. Samples *(lane 3, 4)* were taken and combined with UBE2D3 (10 µM). After a further 10 minutes at 25°C, samples (lane 5, 6) were taken and combined with GST-MYCBP2_{cat} (WT, C4520S, C4520A, C4572S, C4572A, C4520A/C4572S or C4520S/C4572A) (15 µM). The reactions were incubated at 25 °C for 30 seconds and terminated by the addition of 4X LDS loading buffer (either non-reducing or reducing). For Ub~GST-MYCBP2_{cat} C4572S ester bond cleavage, 0.14 N NaOH was added after E3 reaction with E1/E2 mixture for 30 seconds and then further incubated at 37 °C for 20 minutes. The gel was then scanned with a Chemidoc Gel Imaging System (BioRad).

### Multiple turnover amino acid and peptide panel discharge assays

Stock solutions (0.5 M) of amino acids were dissolved in MQ water and pH was adjusted to pH ~8. Peptides of the sequence Ac-EGXGN-NH₂ (X= K, S or T) were obtained from Bio-Synthesis Inc. Stock peptide solutions (200 mM) were dissolved in MQ water and pH was adjusted to pH ~8. An E2 (UBE2D3) charging reaction was carried out in buffer (40 mM Na₂HPO₄-HCl pH 8.0, 150 mM NaCl, 0.5 mM TCEP) containing UBE1 (250-500 nM), UBE2D3 (20 µM), Ub (50 µM), or Cy3B-Ub (25 µM), MgCl₂ (5 mM) and ATP 10 (mM). The reaction was incubated at 37 °C for 15 minutes and then equilibrated to 23 °C for 3 minutes. An equivalent volume of nucleophile sample containing small molecule/peptide nucleophile (100 mM) and GST-MYCBP2 (10 µM) was then added and incubated at 23 °C. Samples were taken at the specified time points and analyzed as described for Tris/glycerol-mediated E2 discharge assay.

Cy3B-Ub was visualized using a Chemidoc Gel Imaging System (Biorad). LC-MS was carried out as described for Tris/glycerol discharge but amino acid substrate samples were quenched by the addition of 2:1 parts quenching solution (75 % acetonitrile, 2 % TFA) and peptide substrate samples were quenched by addition of 1:1 parts quenching solution.

### Multiple turnover E2 discharge panel

E2s were screened for threonine discharge activity with GST-MYCBP2_{cat} as described for the amino acid panel. E2s were also incubated in the presence of threonine but in the absence of GST-MYCBP2_{cat}. These samples provided a reference to distinguish between intrinsic E2~Ub instability and E3-dependent discharge.

### Single turnover E2 mutant discharge by in-gel fluorescence

E2 mutants^{16,17,34-36} (10 µM) were charged with Cy3b-labelled Ub (12.5 µM) in a final volume of 12 µL at 37 °C for 20 minutes then cooled at 23 °C for 3 minutes. E2 recharging was then blocked by the addition of MLN4924 derivative, Compound 1 (25 µM)³⁷, which inhibits E1, and then incubated for a further 15 minutes. The mixture was then mixed with 12 µL of GST-MYCBP2_{cat} (5 µM) and threonine (100 mM) and incubated at 23 °C for the specified time. Analysis was carried out as for multiple turnover assays. To account for intrinsic E2~Ub instability the mean % discharge (n=2) calculated against a parallel incubation where E3 was withheld. Data were plotted using Prism (Graphpad).

### Expression and purification of ARIH1 and UBE3C

ARIH1 residues 1-394 (Dundee clone DU24260) was expressed as an N-terminally GST-tagged fusion protein in BL21 cells. UBE3C residues 641 - 1083 (Dundee Clone DU45301) was expressed as a N-terminally GST-tagged fusion protein in Sf21 cells using the baculovirus infection system.

### Calculation of observed rate constants for E3-substrate dependent single turnover E2~Ub discharge

UBE2D3 or UBE2L3 (5 µM) were charged with Cy3b-labelled Ub (8 µM) in a final volume of 30 µL at 37 °C for 25 minutes then incubated at 23 °C for 3 minutes. Single turnover conditions for E2~Ub discharge were achieved by E1 inhibition with MLN4924 derivative, Compound 1 (25 µM) and then incubated for a further 15 minutes. The mixture was then mixed with 30 µL of MYCBP2_{cat} or ARIH1₁₋₃₉₄ (HHARI) or UBE3C₆₄₁₋₁₀₈₃ (1 µM) and threonine (100 mM) and incubated at 23 °C for the specified time. Samples were quenched with non-reducing 4X LDS loading buffer and resolved by SDS-PAGE (Bis-Tris 4-12 %). The gel was then scanned with a Chemidoc Gel Imaging System (BioRad) and subsequently Coomassie stained. E2~Ub signals were quantified using the Fiji software. Observed rate constants were obtained by fitting reaction progressive curves to a single exponential function using Prism (Graphpad Software).

### MYCBP2 crystallization

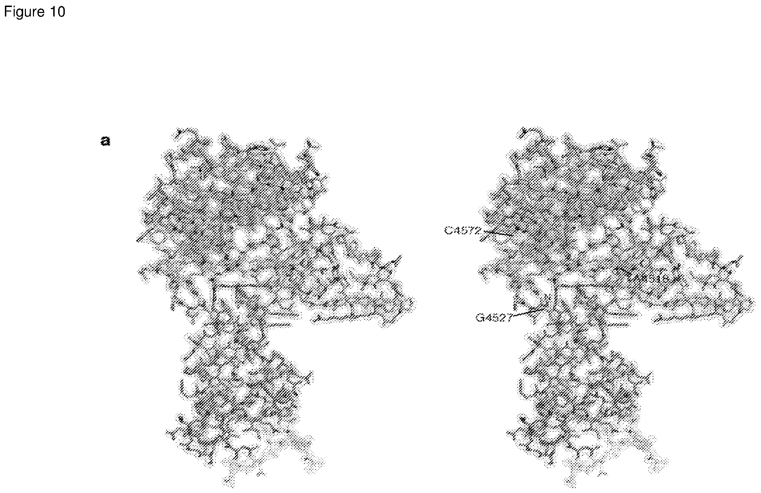

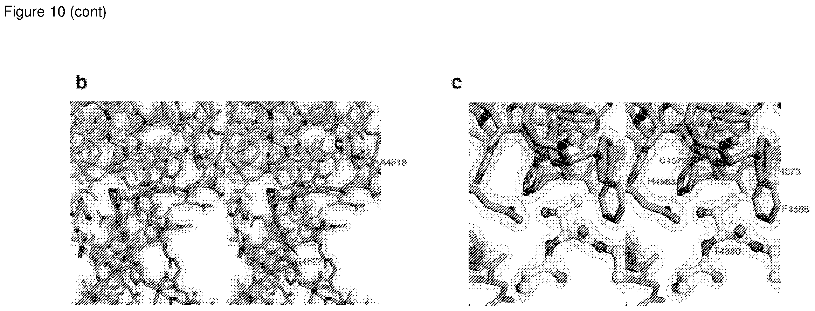

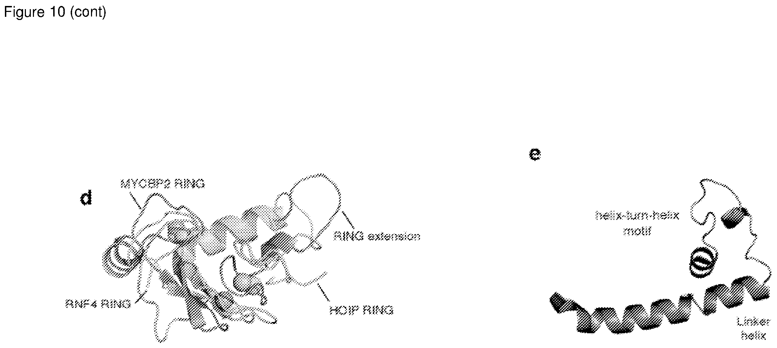

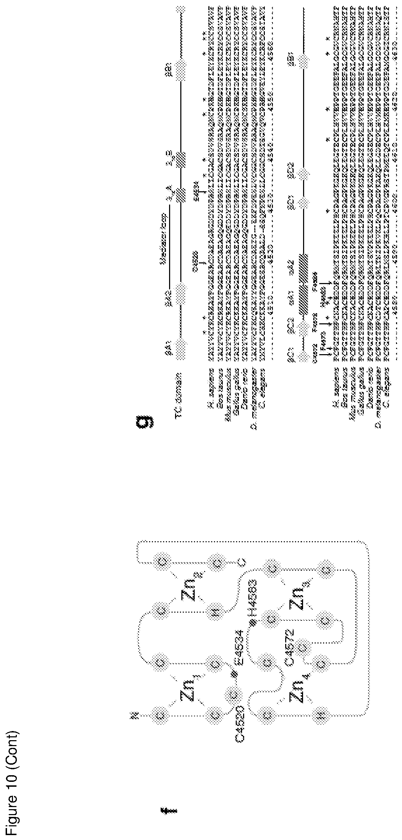

MYCBP2 was expressed as described for untagged protein. After protease cleavage of the tag the protein was further purified by size exclusion chromatography using an ÄKTA FPLC system and a HiLoad 26/600 Superdex 75 pg column (GE Life Sciences). The running buffer consisted of 20 mM HEPES pH 7.4, 150 mM NaCl, 4 mM DTT. Combined fractions were concentrated to 10.4 mg mL⁻¹. Sparse matrix screening was carried out and Bipyrimidal crystals were obtained from the Morpheus screen condition C1 (Molecular Dimensions). A subsequent optimization screen yielded multiple crystals (Buffer system 1 (MES/imidazole) pH 6.7, 23.3 mM Na₂HPO₄, 23.3 mM (NH₄)₂SO₄, 23.3 mM NaNO₃, 18 % PEG500 MME, 9 % PEG20000). A single crystal was soaked in mother liquor and further cryoprotected by supplementation with 5 % PEG400 and frozen in liquid N₂. Data were collected to 1.75 Å at the European Synchrotron Radiation Facility at Beamline ID23-1. Energy was set to the peak value of 9.669 keV (1.2823 Å), as determined by an absorption edge energy scan. A total of 360° were collected with an oscillation range of Ω = 0.1 °. The phase problem was solved by locating 6 Zn²⁺ sites in the anomalous signal and solvent flattening with the SHELX suite. An initial model was built by ARP/wARP³⁸ and subsequently optimized by manual building in COOT³⁹ and refinement with REFMAC5⁴⁰ resulting in the final model with statistics as shown in **Figure 10****.** Final Ramachandran statistics were favored: 95.55 %, allowed: 3.24 %, outliers: 1.21 %.

### Size exclusion chromatography with multi-angle light scattering (SEC-MALS)

SEC-MALS experiments were performed on a Ultimate 3000 HPLC system (Dionex) with an in-line miniDAWN TREOS MALS detector and Optilab T-rEX refractive index detector (Wyatt). In addition, the elution profile of the protein was also monitored by UV absorbance at 280 nm. A Superdex 75 10/300 GL column (GE Life Sciences) was used. Buffer conditions were 50 mM Na₂HPO₄ pH 7.5, NaCl 150 mM, 1.0 mM TCEP and a flow rate of 0.3 mL min⁻¹ was applied. Sample (50 µL, 5.5 mg mL⁻¹) was loaded onto the column with a Dionex autosampler. Molar masses spanning elution peaks were calculated using ASTRA software v6.0.0.108 (Wyatt).

### Mediator loop modelling

Mediator loop residues where built and geometry optimized within the Bioluminate Software (Schrödinger). Side chains were modified within COOT³⁹ and figures were generated with Pymol (Schrödinger). Ramachandran analysis was carried with the RAMPAGE server⁴¹.

### NMNAT2 ubiquitination assay

NMNAT2 (5 µM) was mixed with E1 (500 nM), UBE2D3 (10 µM), MYCBP2_{cat} (10 µM), Ub (50 µM), ATP (10 mM) and made up with 10X pH 7.5 buffer (40 mM Na₂H₂PO₄ pH 7.5, 150 mM NaCl, 5 mM MgCl₂, 0.5 mM TCEP). The reactions were incubated at 37°C for 1 hour and terminated by the addition of 4X LDS loading buffer (either non-reducing or reducing). For base lability test, reactions were supplemented with 0.14 N NaOH and then further incubated at 37 °C for 20 minutes.

### Bioinformatic analysis

Proteins belonging to the RCR family were identified by generalized profile searches. Overall 671 such sequences were identified. The sequences were aligned by profileguided alignment using the pftools package. For identifying representative sequences from various taxa, the Belvu program (Sanger Institute) was used to remove sequences with >80 % identity to other sequences. Truncated and misassembled proteins were removed manually, resulting in 130 representative TC domain sequences.

### Data availability statement

Coordinates have been deposited with the Protein Data Bank (PDB ID 5O6C).

### Results

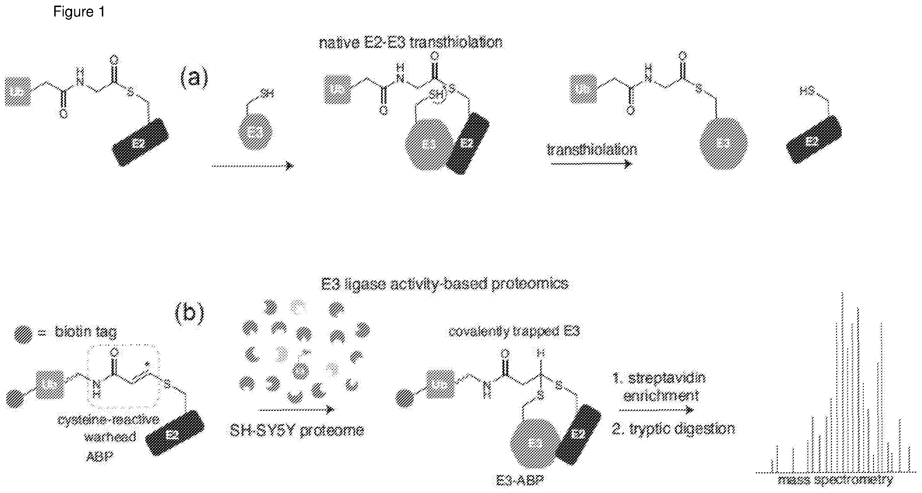

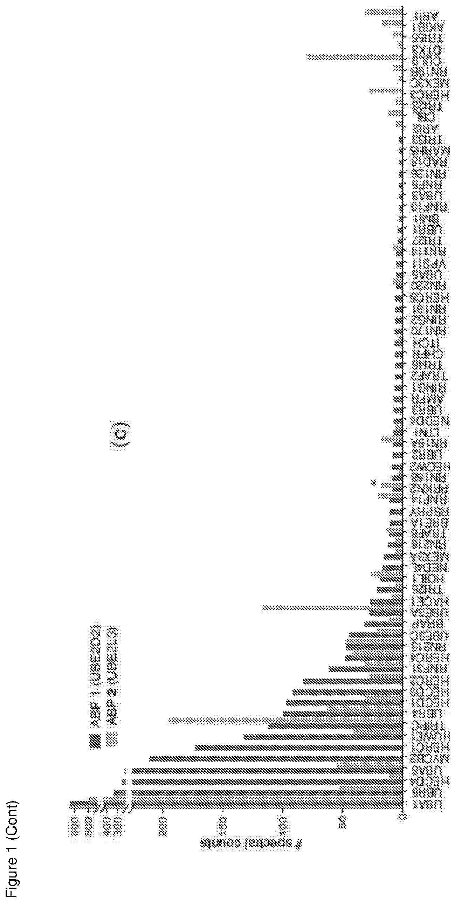

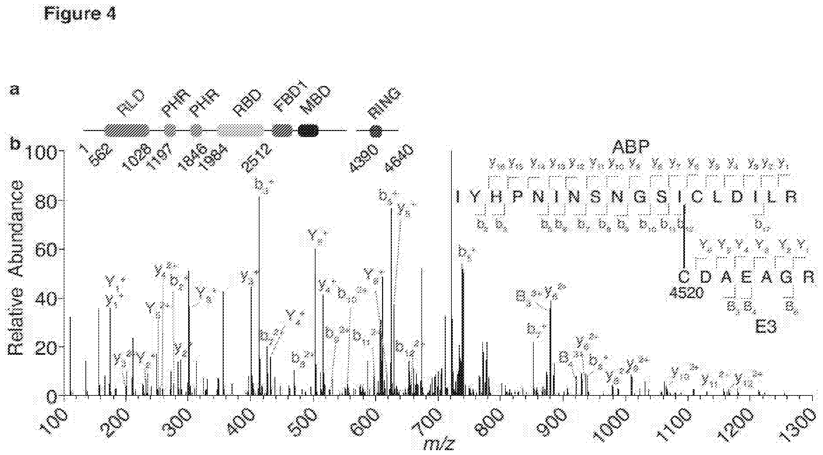

We prepared biotinylated variants of our recently developed activity-based probes (ABPs)⁶ which profile the hallmark transthiolation activity of HECT/RBR E3s **(****Figure 1a** **and** **Figure 2**)⁷. Interfacing the ABP technology with mass spectrometry enabled parallelized profiling of E3 activity in neuroblastoma SH-SY5Y cell extracts^{8,9} **(****Figure 1b****)**. E3s were filtered using criteria ensuring signals for at least a subset of detected E3s correlated with E3 activity and/or abundance **(****Figure 3****).** Profiling of -80% of the -50 known HECT/RBR E3s was achieved but unexpectedly, 33 RING E3s, devoid of HECT or RBR ancillary domains, were also enriched **(****Figure 1c****-e).** To explore the possibility that hitherto undiscovered RING-linked E3s were being labelled we focused on MYCBP2/Phr1 (Myc-binding protein 2; PAM/Highwire/Rpm-1) **(****Figure 1c****).** MYCBP2 is a large 0.5 MDa neuron-associated protein which contains a C-terminal RING domain **(****Figure 4a****),** and is involved in a range of cellular processes including regulation of nervous system development and axon degeneration¹⁰⁻¹².

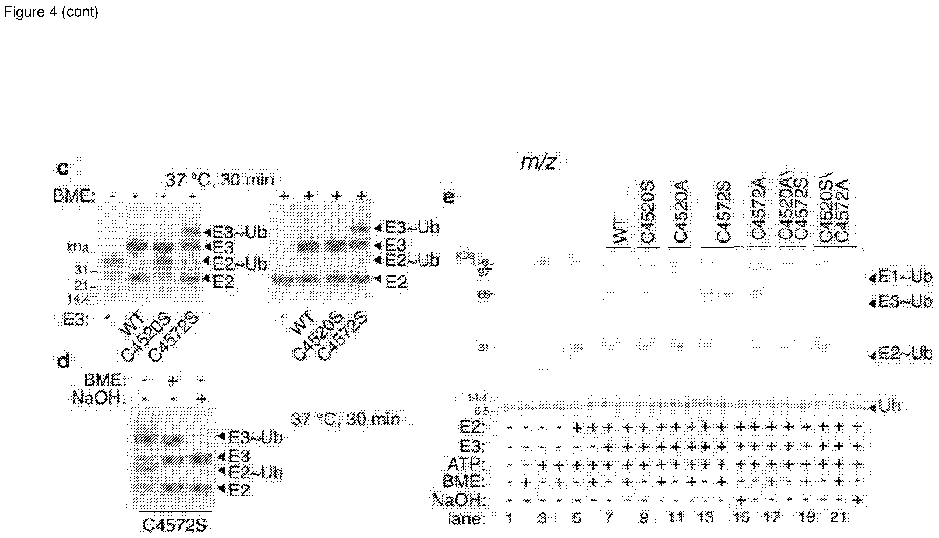

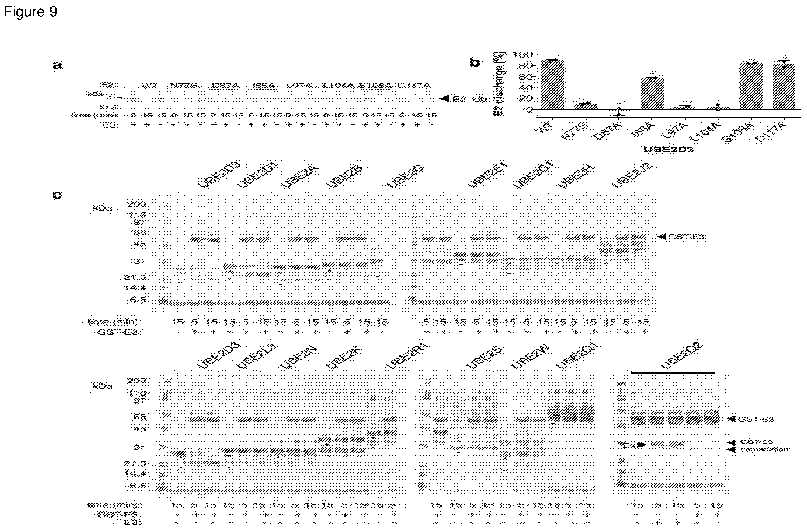

A recombinant C-terminal version of MYCBP2 encompassing the RING domain (residues 4378-4640; MYCBP2_{cat}; **Figure 4a****)** and an uncharacterized C-terminal cysteine-rich region underwent robust ABP labelling with an efficiency comparable to that of E3s known to demonstrate transthiolation activity^{7,13} **(****Figure 5a****).** To map the putative catalytic cysteine we used a combination of ABP-based profiling and ABP-crosslinking MS⁷ **(****Figure 2b****,** **Figure 5b, c****,).** Data were in support of C4520 being a putative catalytic residue. We next assayed wild type (WT) E3 activity but were unable to detect autoubiquitination or free Ub chain formation. However, we observed rapid E3-dependent discharge of Ub from E2~Ub suggestive of the presence of an unknown small molecule nucleophilic acceptor **(****Figure 2c****).** Liquid chromatography-mass spectrometry (LC-MS) analysis revealed that Ub was being quantitatively converted into two species with masses corresponding to condensation products with *tris*(hydroxymethyl)aminomethane (Tris) and glycerol (8639 and 8668 Da), both of which were present in our assay buffer at routinely employed concentrations of 50 mM and -65 mM, respectively. Due to the common hydroxy functionality within these nucleophiles, MYCBP2 appeared to have esterification activity **(****Figure 9a**, b). Activity was found to be dependent on C4520, consistent with it forming a thioester-linked E3~Ub intermediate^{4,5} (**Figure 4c**).

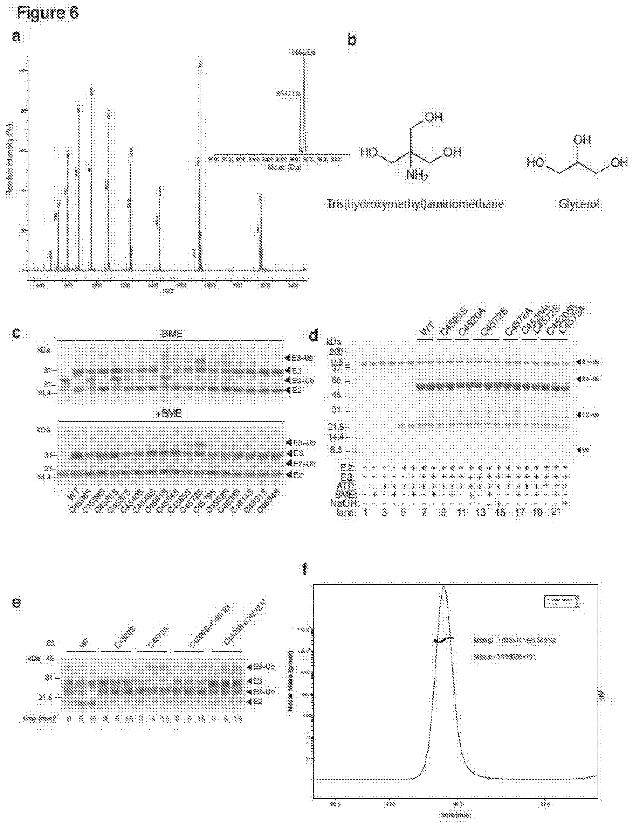

Unexpectedly, a MYCBP2 C4572S mutant retained activity yet formed a discrete mono Ub adduct that was resistant to thiolysis but reversible after base treatment (**Figure 4c**, **d** and **Figure 6c****)**⁵. A possibility was the mutated S4572 residue was contributing to catalysis via formation of a less transient (oxy)ester-linked intermediate between Ub and S4572 that retained the ability to modify substrate. We hypothesized that C4520 and C4572 were both catalytic residues that functioned in tandem by relaying Ub from one cysteine to the other through an intramolecular transthiolation reaction. To test this relay mechanism, we carried out gel-based thioester/ester trapping assays¹⁴ **(****Figure 4e** **and** **Figure 6d****)** and observed a thiol-sensitive Ub adduct on WT MYCBP2_{cat} which was not observed with the C4520S mutant **(****Figure 4e****).** Consistent with earlier experiments (**Figure 4c**), C4572S underwent adduct formation that was thiol-resistant but base labile **(****Figure 4e****).** Thus, if a transient thioester intermediate was being formed between Ub and C4520, then an unreactive C4572A mutant should stabilize it. Indeed, thiol-sensitive adduct formation was increased on a C4572A mutant relative to that of the wild type and was presumably linked via a thioester bond (Figure 4e). Adduct formation was not detected with a C4520A/C4572S double mutant in support of it being linked to the C4520 residue **(****Figure 4e****).** In the absence of direct demonstration of Cys-to-Cys Ub transfer we cannot formally exclude other possibilities. However, the existing data are consistent with the essential cysteines functioning in a relay mechanism. Mutational analysis and size exclusion chromatography-multi angle light scattering (SEC-MALS) of MYCBP2_{cat} **(****Figure 6e, f**) suggests the proposed relay mechanism requires both essential cysteines be in the same molecule, consistent with an intramolecular relay mechanism.

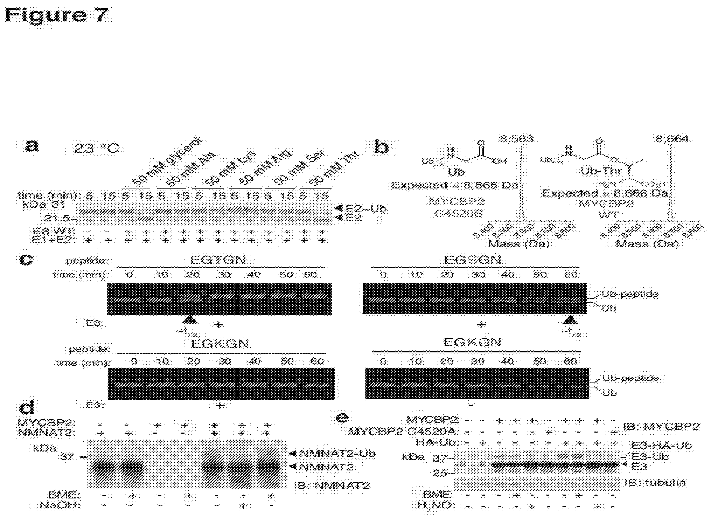

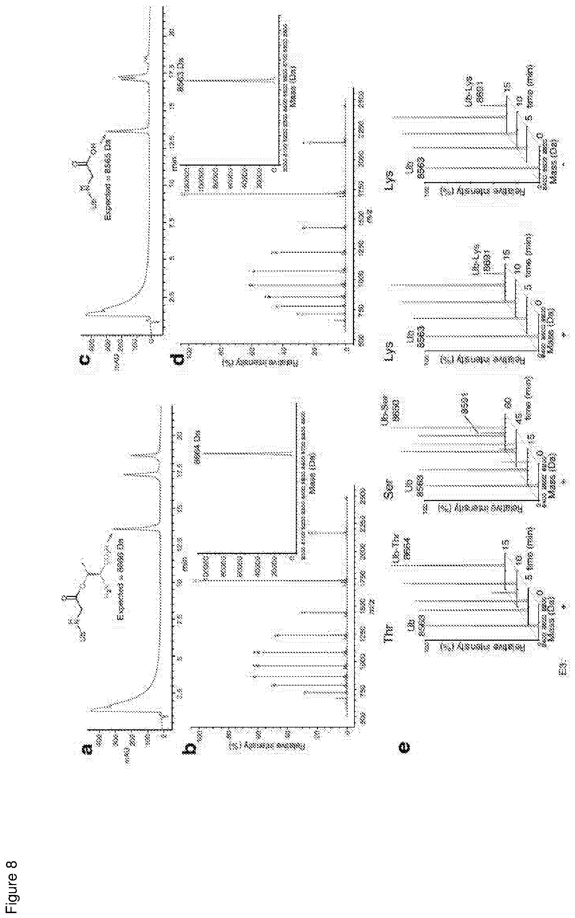

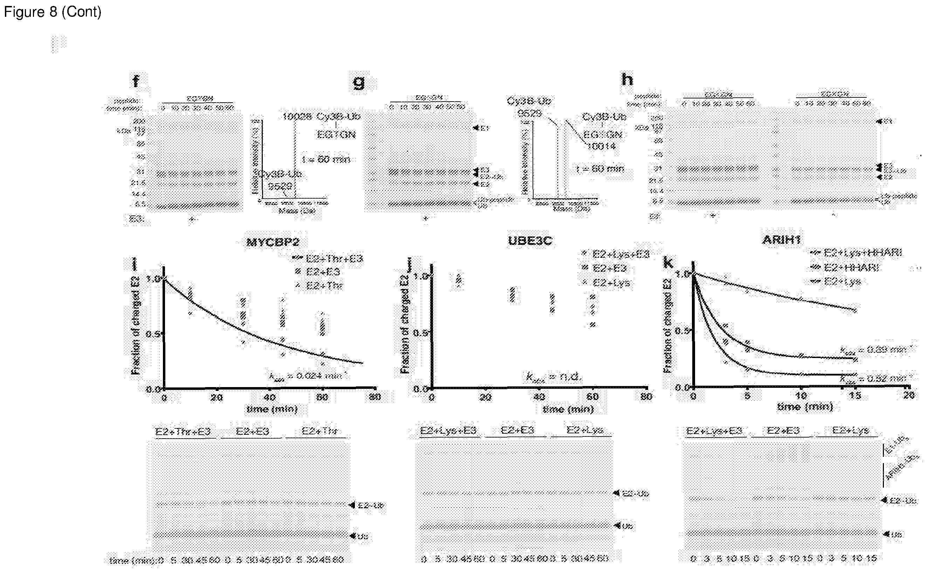

In light of the observed esterification activity we attempted to identify the amino acid substrate of MYCBP2 by screening a panel of amino acids⁵ where discharge activity was strikingly enhanced towards threonine. Product formation was dependent on C4520 **(****Figure 7a, b** and **Figure 8a****-d).** MS-based quantification indicated ~10-fold selectivity for threonine over serine **(****Figure 8e****).** Although a low level of lysine modification was observed, this was independent of MYCBP2_{cat}⁵. Threonine selectivity (3-fold ) was also maintained in a peptide context **(****Figure 7c** and **Figure 8f****-h).** Furthermore, basal ubiquitination of a lysine peptide was partially inhibited in the presence of MYCBP2_{cat} underscoring its lack of lysine activity **(****Figure 7c****).** Taken together, our experiments revealed that MYCBP2 is a novel class of E3 enzyme that operates via two essential cysteines, promotes Ub modification of hydroxyl groups, and esterifies threonine with Ub with selectivity over serine. As MYCBP2 uses a novel mechanism we termed it a RING-Cys-Relay (RCR) E3. We benchmarked the catalytic efficiency of MYCBP2 threonine esterification activity and found it to fall between that of well-characterized HECT and RBR E3 lysine aminolysis activity^{5,15} (**Figure 8i-k**). E2 mutational analysis^{5,16-19}, was in further support of MYCBP2_{cat} being a novel class of E3 **(****Figure 9a**, **b** and Methods). To ascertain functional E2 partners, 17 E2s were tested but only UBE2D1, UBE2D3 and UBE2E1 demonstrated robust activity **(****Figure 9c****).**

MYCBP2 promotes Wallerian axon degeneration through destabilization of Nicotinamide Mononucleotide Adenyltransferase (NMNAT2)²⁰. We next tested whether MYCBP2_{cat} can ubiquitinate NMNAT2 by esterification *in vitro* **(****Figure 7d****).** Despite containing 13 lysine residues, NMNAT2 underwent hydroxide labile but thiol resistant ubiquitination, demonstrating that MYCBP2 can target hydroxy residues within one of its putative substrates²⁰. Cellular substrate recognition is mediated by a Skp1/Fbox45 substrate receptor co-complex that binds to a site -1940 residues N-terminal to the MYCBP2_{cat} region **(****Figure 7a**)²¹. NMNAT2 also undergoes palmitoylation and rapid axonal transport²² making reconstitution and cellular study of its ubiquitination extremely challenging. However, to establish whether MYCBP2_{cat} retains non-lysine activity in cells we looked at its autoubiquitination after transient transfection into human embryonic kidney 293 cells. Base-labile (but thiol-resistant) ubiquitination was observed that was dependent on C4520 **(****Figure 7e****).** This demonstrates that MYCBP2 can retain specificity for hydroxy amino acids in cells and that this activity remains dependent on the upstream catalytic residue we implicate with a Ub relay mechanism.

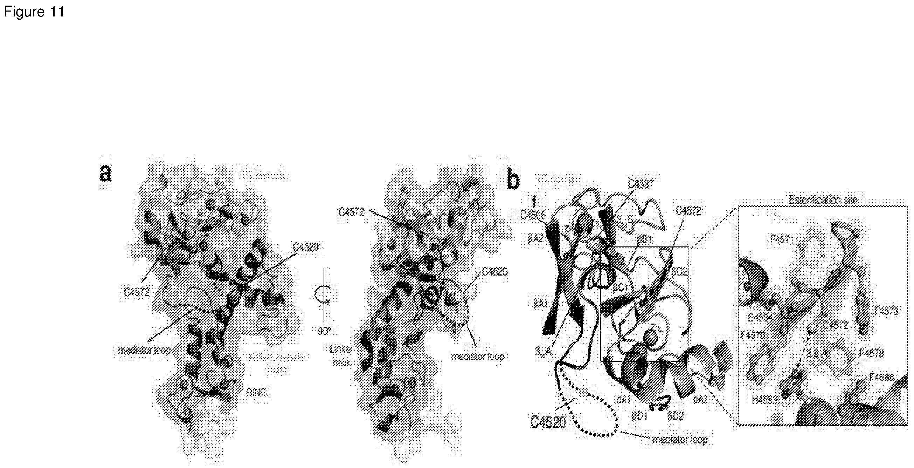

To further validate the RING-Cys-relay model and the serine/threonine activity we crystallized MYCBP2_{cat} (residues 4378-4640) and solved a crystal structure to a resolution 1.75 Å **(Table 1 and** **Figure 10a****-c).** Residues 4388-4441 at the N-terminus correspond to the predicted cross-brace C3H2C3 RING domain **(****Figure 4a** **and** **Figure 10d****).** Following the RING domain is a long α-helix (4447-4474) that leads into small helix-turn-helix motif (residues 4475-4500) **(****Figure 11a** **and** **Figure 10e****)** and further C-terminal is a structurally unprecedented globular domain that binds four Zn ions (residues 4501-4638) **(****Figure 11b** **and** **Figure 10f, g**). Since this domain also contains the two essential catalytic residues we term it the Tandem Cysteine (TC) domain. Between the βA2 strand and helix 3₁₀A is an unstructured region (4519-4526) which projects out to the side of the core Zn-binding fold. The upstream C4520 residue resides within this unstructured region, which together with flanking residues, forms a mobile region we term the mediator loop. The Zn coordination configuration (C5HC7HC2) of the TC domain is semi-contiguous and does not adopt cross-brace architecture **(****Figure 10c****).**

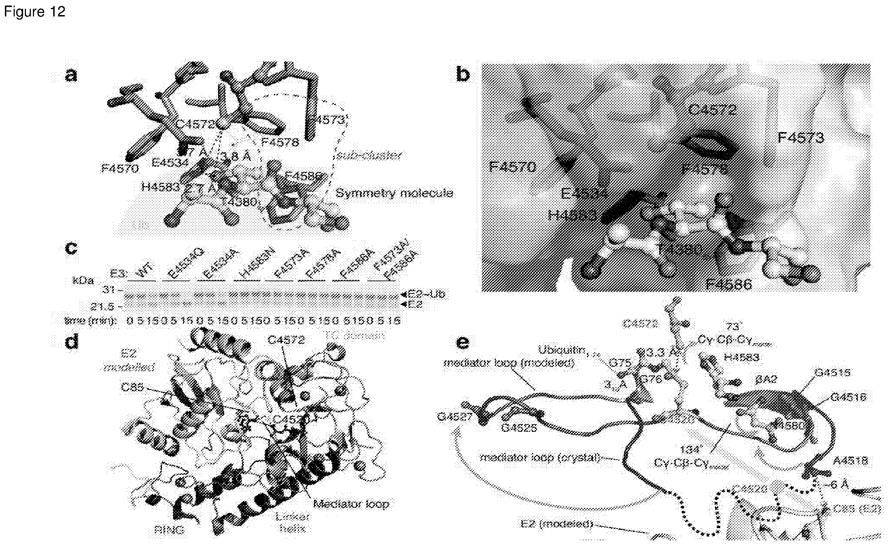

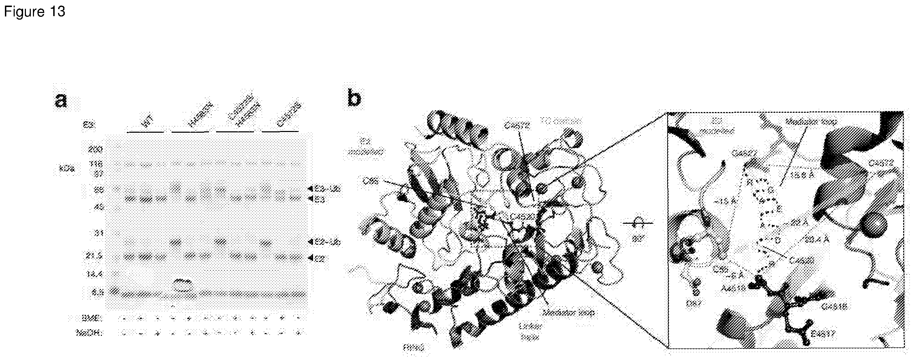

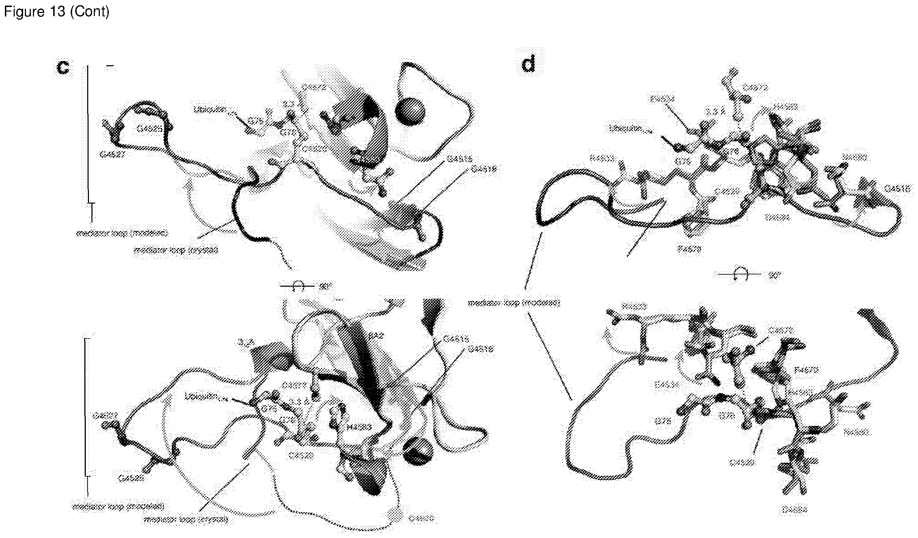

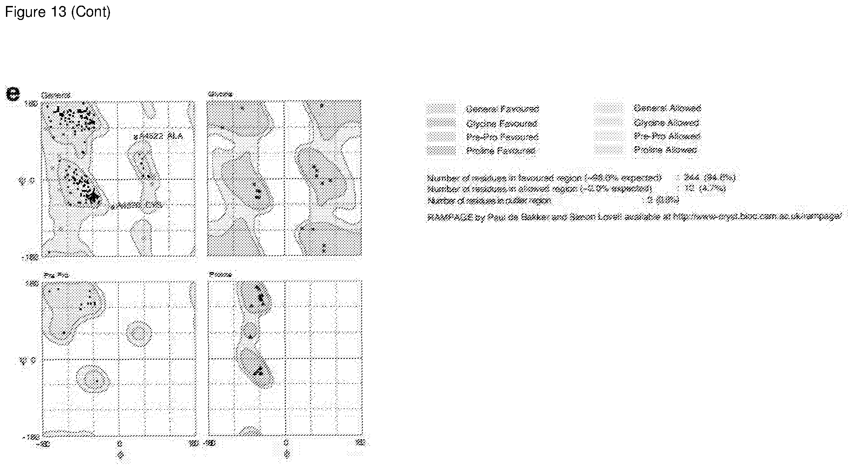

Crystal packing revealed that T4380, within the N-terminus of a symmetry-related MYCBP2_{cat} molecule (T4380_{sym}), was placed proximal to the esterification site where it forms a number of substrate-like interactions **(****Figure 12a** **& b).** Firstly, the β-hydroxy group of T4380_{sym} complements E4534 and H4583 and forms a potential triad **(****Figure 12a****).** Thus the β-oxygen atom of T4380_{sym} appears to be primed for deprotonation and nucleophilic attack. A catalytically productive electrophilic center is the C-terminus of Ub when thioester-linked to C4572. Even though this Ub molecule is absent in our structure, the C4572 sulfur atom is 3.8 Å away from the β-oxygen atom of T4380_{sym}. Thus, the structure appears to accurately reflect a catalytic intermediate poised to undergo threonine ubiquitination by esterification of its β-hydroxy group **(****Figure 12a****).** Furthermore, a sub-cluster of Phe residues (F4573, F4578 and F4586), proximal to the β-methyl group of T4380_{sym}, **(****Figure 12a** **and b)** forms a well-defined hydrophobic pocket which the T4380_{sym} β-methyl group docks into and appears to be a positive selectivity determinant for the threonine side chain. The proposed roles of these residues were validated in threonine discharge assays **(****Figure 12c****).** An H4583N mutation abolished activity consistent with a role as a general base. The H4583N mutant also underwent enhanced, thiol-sensitive, Ub adduct formation in accordance with the anticipated defect in rendering substrate nucleophiles reactive towards the C4572 thioester **(****Figure 13a****).** Conservative perturbation of the phenylalanine cluster also markedly reduced threonine discharge activity **(****Figure 5c****).** Perturbation of E4534 did not reduce activity hence its precise role remains unclear.

Conservation of RING domain binding to E2^{16-18,23} permitted the modelling of an E2-RCR E3 ligase complex which was geometrically compatible with transthiolation between E2~Ub and MYCBP2_{cat} C4520 (**Figure 5d** and **Figure 13b****).** To simulate the conformation required for subsequent Ub relay to C4572 we modelled the missing mediator loop residues with a GlyGly dipeptide thioester linked to C4520, representative of the Ub C-terminus that would be transferred via transthiolation with E2~Ub if our relay model was valid **(****Figure 13c****-e).** In support of this mechanism the carbonyl C atom of the Ub thioester could be positioned in proximity (3.3 Å) of the C4572 sulfhydryl sulfur atom. To adopt this conformation minor twisting of a GlyGly motif (residues 4515-4516) at the tip of the βA2 strand was necessary. Clashes were observed between mediator loop residues further C-terminal with R4533, E4534, N4580, H4583 and D4584 but these could be largely relieved by rotations of their sidechains into available space. As ordered loop residues 4527-4531 required a significant displacement to generate the model, we speculate that the mobile mediator loop region would span residues 4515-4531. As C4520, which resides within this mobile structural element, needs to be engaged by the E2 active site²⁴ this might account for the uncharacterized E2 residue requirements. An explanation for the inability to render the S4520 mutant catalytic in earlier experiments is its dynamic nature and the absence of a general base which could suppress the *pKₐ* of the otherwise fully protonated S4520 side chain. Hence native C4520 catalytic activity is likely to arise from the sulfhydryl groups intrinsic nucleophilicity **(****Figure 13a****).**

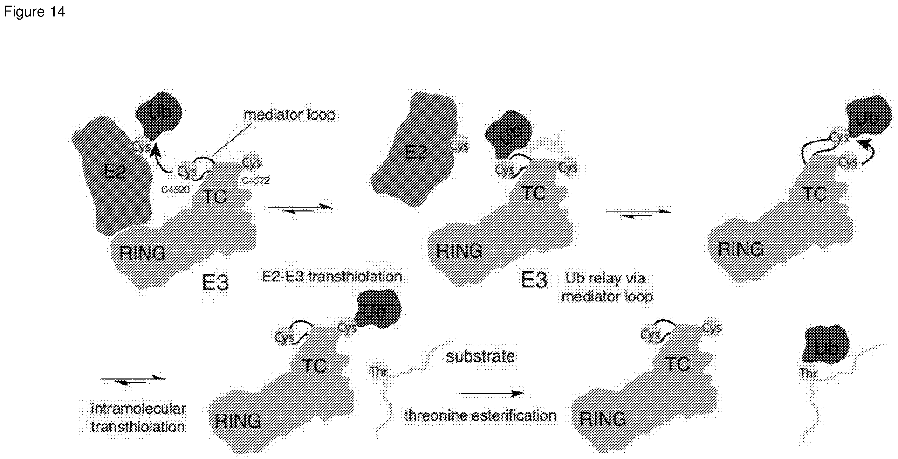

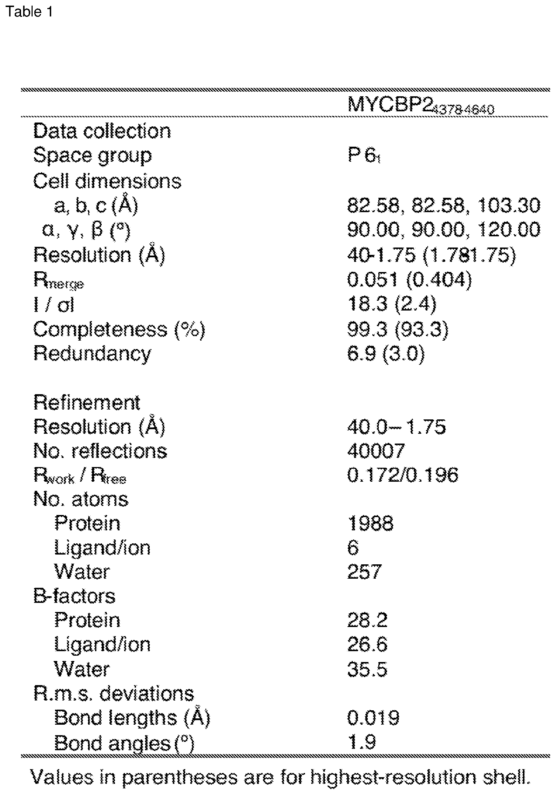

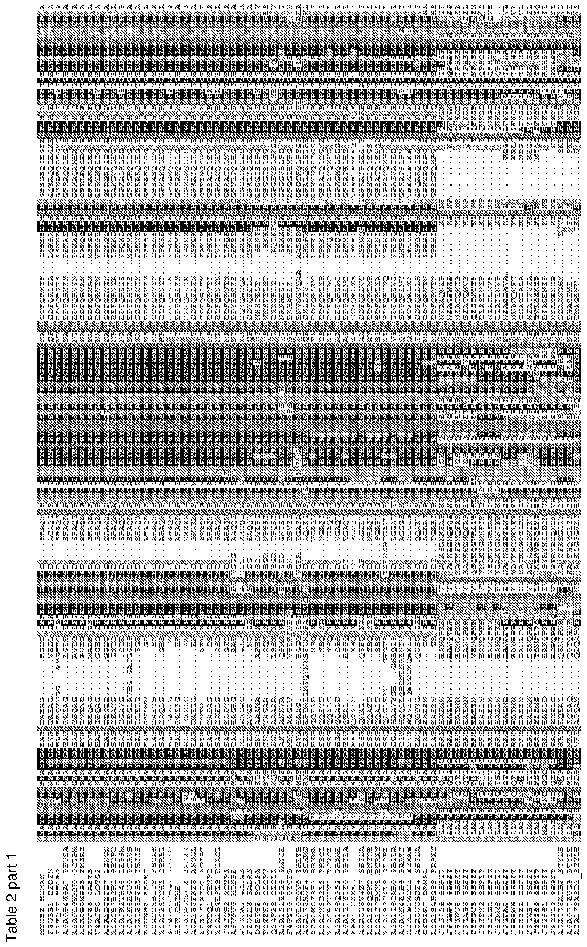

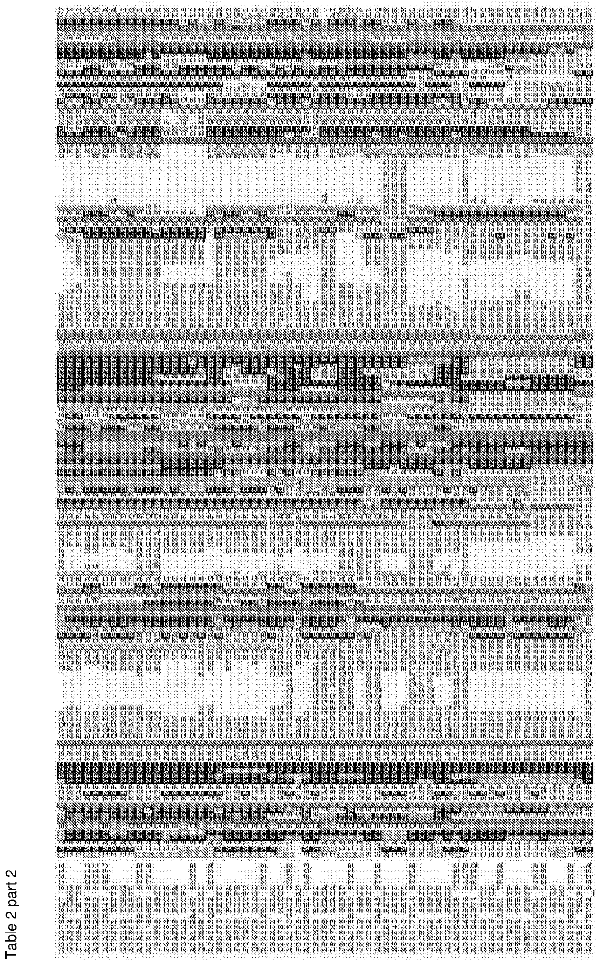

Although non-lysine ubiquitination has been reported²⁵⁻²⁷, a human E3 ligase that preferentially carries out this function has remained elusive. Our characterization of the novel RCR E3 ligase found in MYCBP2 suggests that ubiquitination by esterification is intrinsic to higher eukaryotes and may be a regulator of synapse development and axon degradation. Furthermore, non-protein Ub substrates (e.g. lipids, carbohydrates) have not been reported but considering the high esterification activity of MYCBP2 towards small molecule hydroxy compounds, this remains a possibility. It is not immediately clear why the proposed relay (**Figure 14a**) mechanism would have evolved. However, transthiolation is a cofactor independent process providing a facile means to shuttle Ub throughout the ubiquitin system¹. We speculate that on steric grounds, direct E2-E3 transthiolation with the structurally rigid and highly conserved E2 ubiquitin conjugating domain (Ubc)²⁸, and serine/threonine activity, are mutually exclusive at the esterification site and evolution of the mediator loop addresses this compatibility issue. Bioinformatic analysis revealed that MYCBP2 orthologues are found in virtually all animals but human homologues are unlikely to exist **(Table 2).**

### Discussion

Stabilization of NMNAT2 through MYCBP2 inhibition is a promising therapeutic strategy for mitigating neuron damage after injury and administration of chemotherapeutics^{10,29,30} and slowing the progression of a range of neurodegenerative diseases including Alzheimer's and Parkinson's²⁹. The delineation of this apparent Ub relay mechanism and the structural characterization of the molecular machinery responsible opens up new medical potential for treating a range of neurological conditions.

### References

1 Hershko, A. & Ciechanover, A. The ubiquitin system. Annu Rev Biochem 67, 425-479, (1998).
2 Deshaies, R. J. & Joazeiro, C. A. RING domain E3 ubiquitin ligases. Annu Rev Biochem 78, 399-434, (2009).
3 Huibregtse, J. M., Scheffner, M., Beaudenon, S. & Howley, P. M. A family of proteins structurally and functionally related to the E6-AP ubiquitin-protein ligase. Proc Natl Acad Sci U S A 92, 2563-2567 (1995).
4 Scheffner, M., Nuber, U. & Huibregtse, J. M. Protein ubiquitination involving an E1-E2-E3 enzyme ubiquitin thioester cascade. Nature 373, 81-83, (1995).
5 Wenzel, D. M., Lissounov, A., Brzovic, P. S. & Klevit, R. E. UBCH7 reactivity profile reveals parkin and HHARI to be RING/HECT hybrids. Nature 474, 105-108, (2011).
6 Hewings, D. S., Flygare, J. A., Bogyo, M. & Wertz, I. E. Activity-based probes for the ubiquitin conjugation-deconjugation machinery: new chemistries, new tools, and new insights. FEBS J 284, 1555-1576, (2017).
7 Pao, K. C. et al. Probes of ubiquitin E3 ligases enable systematic dissection of parkin activation. Nat Chem Biol 12, 324-331, (2016).
8 Niphakis, M. J. & Cravatt, B. F. Enzyme inhibitor discovery by activity-based protein profiling. Annu Rev Biochem 83, 341-377, (2014).
9 Borodovsky, A. et al. Chemistry-based functional proteomics reveals novel members of the deubiquitinating enzyme family. Chem Biol 9, 1149-1159 (2002).
10 Zhen, M., Huang, X., Bamber, B. & Jin, Y. Regulation of presynaptic terminal organization by C. elegans RPM-1, a putative guanine nucleotide exchanger with a RING-H2 finger domain. Neuron 26, 331-343 (2000).
11 Wan, H. I. et al. Highwire regulates synaptic growth in Drosophila. Neuron 26, 313-329 (2000).
12 Grill, B., Murphey, R. K. & Borgen, M. A. The PHR proteins: intracellular signaling hubs in neuronal development and axon degeneration. Neural Dev 11, 8 (2016).
13 Byrne, R., Mund, T. & Licchesi, J. Activity-Based Probes for HECT E3 ubiquitin ligases. Chembiochem, 18, 1415-1427 (2017).
14 Stieglitz, B., Morris-Davies, A. C., Koliopoulos, M. G., Christodoulou, E. & Rittinger, K. LUBAC synthesizes linear ubiquitin chains via a thioester intermediate. EMBO Rep 13, 840-846, (2012).
15 You, J. & Pickart, C. M. A HECT domain E3 enzyme assembles novel polyubiquitin chains. J Biol Chem 276, 19871-19878 (2001).
16 Plechanovova, A., Jaffray, E. G., Tatham, M. H., Naismith, J. H. & Hay, R. T. Structure of a RING E3 ligase and ubiquitin-loaded E2 primed for catalysis. Nature 489, 115-120, (2012).
17 Dou, H., Buetow, L., Sibbet, G. J., Cameron, K. & Huang, D. T. BIRC7-E2 ubiquitin conjugate structure reveals the mechanism of ubiquitin transfer by a RING dimer. Nat Struct Mol Biol 19, 876-883, (2012).
18 Lechtenberg, B. C. et al. Structure of a HOIP/E2~ubiquitin complex reveals RBR E3 ligase mechanism and regulation. Nature 529, 546-550, (2016).
19 Dove, K. K. et al. Structural Studies of HHARI/UbcH7~Ub Reveal Unique E2~Ub Conformational Restriction by RBR RING1. Structure 25, 890-900 e895, (2017).
20 Xiong, X. et al. The Highwire ubiquitin ligase promotes axonal degeneration by tuning levels of Nmnat protein. PLoS Biol 10, e1001440, (2012).
21 Babetto, E., Beirowski, B., Russler, E. V., Milbrandt, J. & DiAntonio, A. The Phr1 ubiquitin ligase promotes injury-induced axon self-destruction. Cell Rep 3, 1422-1429, (2013).
22 Milde, S., Gilley, J. & Coleman, M. P. Subcellular localization determines the stability and axon protective capacity of axon survival factor Nmnat2. PLoS Biol 11, e1001539, (2013).
23 Zheng, N., Wang, P., Jeffrey, P. D. & Pavletich, N. P. Structure of a c-Cbl-UbcH7 complex: RING domain function in ubiquitin-protein ligases. Cell 102, 533-539 (2000).
24 Olsen, S. K. & Lima, C. D. Structure of a ubiquitin E1-E2 complex: insights to E1-E2 thioester transfer. Mol Cell 49, 884-896, (2013).
25 Cadwell, K. & Coscoy, L. Ubiquitination on nonlysine residues by a viral E3 ubiquitin ligase. Science 309, 127-130, (2005).
26 Shimizu, Y., Okuda-Shimizu, Y. & Hendershot, L. M. Ubiquitylation of an ERAD substrate occurs on multiple types of amino acids. Mol Cell 40, 917-926, (2010).
27 Wang, X., Herr, R. A. & Hansen, T. H. Ubiquitination of substrates by esterification. Traffic 13, 19-24, (2012).
28 Alpi, A. F., Chaugule, V. & Walden, H. Mechanism and disease association of E2-conjugating enzymes: lessons from UBE2T and UBE2L3. Biochem J 473, 3401-3419, (2016).
29 Conforti, L., Gilley, J. & Coleman, M. P. Wallerian degeneration: an emerging axon death pathway linking injury and disease. Nat Rev Neurosci 15, 394-409, (2014).
30 Ali, Y. O., Bradley, G. & Lu, H. C. Screening with an NMNAT2-MSD platform identifies small molecules that modulate NMNAT2 levels in cortical neurons. Sci Rep 7, 43846, (2017).
31 Stieglitz, B. et al. Structural basis for ligase-specific conjugation of linear ubiquitin chains by HOIP. Nature 503, 422-426, (2013).
32 Stanley, M. et al. Orthogonal thiol functionalization at a single atomic center for profiling transthiolation activity of E1 activating enzymes. ACS Chem Biol 10, 1542-1554, (2015).
33 Yang, B. et al. Identification of cross-linked peptides from complex samples. Nat Methods 9, 904-906, (2012).
34 Pruneda, J. N. et al. Structure of an E3:E2~Ub complex reveals an allosteric mechanism shared among RING/U-box ligases. Mol Cell 47, 933-942, (2012).
35 Wu, P. Y. et al. A conserved catalytic residue in the ubiquitin-conjugating enzyme family. EMBO J 22, 5241-5250, (2003).
36 Yunus, A. A. & Lima, C. D. Lysine activation and functional analysis of E2-mediated conjugation in the SUMO pathway. Nat Struct Mol Biol 13, 491-499, (2006).
37 Brownell, J. E. et al. Substrate-assisted inhibition of ubiquitin-like proteinactivating enzymes: the NEDD8 E1 inhibitor MLN4924 forms a NEDD8-AMP mimetic in situ. Mol Cell 37, 102-111, (2010).
38 Langer, G., Cohen, S. X., Lamzin, V. S. & Perrakis, A. Automated macromolecular model building for X-ray crystallography using ARP/wARP version 7. Nat Protoc 3, 1171-1179, (2008).
39 Emsley, P., Lohkamp, B., Scott, W. G. & Cowtan, K. Features and development of Coot. Acta Crystallogr D Biol Crystallogr 66, 486-501, (2010).
40 Murshudov, G. N. et al. REFMAC5 for the refinement of macromolecular crystal structures. Acta Crystallogr D Biol Crystallogr 67, 355-367, (2011).
41 Lovell, S. C. et al. Structure validation by Cα geometry: phi, psi and Cβ deviation. Proteins 50, 437-450, (2003).
42 Jinek, M., et al. (2012) Science, 337, 816-821.
43 Overballe-Petersen, S., et al. (2013) Proc. Natl. Acad. Sci. U.S.A. 110,19860-19865.
44 Gong, C., et al. (2005) Nat. Struct. Mol. Biol. 12, 304-312.
45 Davis, L., Maizels, N. (2014) Proc. Natl. Acad. Sci. U S A, 111, E924-932.
46 Ran, F.A., et al. (2013) Cell, 154, 1380-1389.
47 Qi, L.S., et al. (2013) Cell, 152, 1173-1183.
48 Gasiunas, G., et al. (2012) Proc. Natl. Acad. Sci. U S A, 109, E2579-2586.
49 Maede, M.L., et al. (2013) Nat. Methods, 10, 977-979
50 Gilbert, L.A., et al. (2013) Cell, 154, 442-451.
51 Hu, J., et al. (2014) Nucleic Acids Res. doi:10.1093/nar/gku109.
52 Perez-Pinera, P., et al. (2013) Nat. Methods, 10, 239-242.
53 Chen, B., et al. (2013) Cell, 155, 1479-1491.
54 Seung, W., et al. (2014) Genome Res. 24, 132-141.
55 Miller, J.C., et al. (2011). Nat. Biotechnol. 29, 143-148.
56 Mussolino, C., et al. (2011). Nucleic Acids Res. 39, 9283-9293.
57 Maeder, M.L., et al. (2008) Mol. Cell, 31, 294-301.
58 Hwang, W.Y., et al. (2013) PLoS One, 8:e68708.
59 Feng, Z., et al. (2013) Cell Res. 23, 1229-1232.
60 Mali, P., et al. (2013) Science, 339, 823-826.
61 Zhou, J., etal. (2014) FEBS J. doi:10.1111/febs.12735.
62 Fu, Y., et al. (2013) Nat. Biotechnol. 31, 822-826.
63 Fu, Y., et al. (2014) Nat Biotechnol. doi: 10.1038/nbt.2808.
64 Hsu, P.D., et al. (2013) Nat. Biotechnol. 31, 827-832.
65 Sander, J.D., et al. (2007) Nucleic Acids Res. 35, W599-605.
66 Sander, J.D., et al (2010) Nucleic Acids Res. 38, W462-468.

## Claims

1. A method for identifying a modulator of MYCBP2 esterification activity towards hydroxy containing substrates, the method comprising:
a) contacting MYCBP2, or an orthologue, or mutant, or fragment thereof comprising at least the active site of the MYCBP2 native protein with a test substance, wherein the active site comprises two catalytic cysteines, wherein the catalytic cysteines are C4520 and C4572 (the numbering according to the native full-length MYCBP2 canonical isoform disclosed in the description), wherein C4520 resides within a mediator loop region represented by residues 4515-4531, wherein transthiolation between E2 conjugating enzyme (E2) and one of the cysteine residues leads to intramolecular relay of ubiquitin to the other cysteine residue and then to its substrate;
b) providing a probe with a hydroxy group which is capable of interacting with C4520, C4572 and/or the mediator loop region of active MYCBP2, or the orthologue, or mutant or fragment thereof; and
c) detecting whether or not the level of interaction of the probe with MYCBP2, the orthologue, or mutant or fragment thereof is modulated as compared to the level of interaction in the absence of the test substance.

2. The method according to claim 1, wherein the probe comprises a non-endogenous substrate of active MYCBP2.

3. The method according to claim 1 or claim 2, wherein the modulator is an inhibitor and step c) comprises detecting whether or not the level of interaction of the probe with MYCBP2, or the orthologue, or mutant, or fragment thereof is reduced as compared to the level of interaction in the absence of the test substance.

4. The method according to any preceding claim, wherein the probe is conjugated to a surface.

5. The method according to claim 1, wherein the probe comprises a peptide, optionally, wherein the probe comprises threonine or serine.

6. The method according to claim 1, wherein the probe comprises a small molecule having a molecular weight of no more than 2kDa.

7. The method according to claim 5 or claim 6, wherein the probe comprises tris, glycerol or HEPES.

8. The method according to any preceding claim, wherein the orthologue is selected from mouse Phr1, zebrafish Esrom/phr1, Drosophila Highwire and C.elegans RPM-1.

9. The method according to any preceding claim, wherein the test substance comprises a peptide, a small molecule, an aptamer, an antibody or an antibody fragment.

10. The method according to any preceding claim, wherein the probe comprises a label.

11. The method according to claim 10, wherein the label comprises an affinity tag.

12. The method according to any preceding claim, wherein the method comprises contacting a fragment of MYCBP2, wherein the fragment comprises residues 4390 to 4572 of MYCBP2.

## Patentansprüche

1. Verfahren zur Identifizierung eines Modulators von MYCBP2-Veresterungsaktivität gegenüber hydroxyhaltigen Substraten, wobei das Verfahren Folgendes umfasst:
a) In-Kontakt-Bringen von MYCBP2, oder eines Orthologs, oder eines Mutanten, oder eines Fragments davon, umfassend mindestens das aktive Zentrum des nativen MYCBP2-Proteins, mit einer Testsubstanz, wobei das aktive Zentrum zwei katalytische Cysteine umfasst, wobei die katalytischen Cysteine C4520 und C4572 sind (wobei die Nummerierung gemäß der nativen MYCBP2 kanonischen Isoform in voller Länge in der Beschreibung offengelegt wird), wobei C4520 innerhalb einer Mediator-Schleifenregion befindlich ist, dargestellt durch die Rückstände 4515 bis 4531, wobei eine Tansthiolation zwischen dem E2 konjugierenden Enzym (E2) und einem der Cysteinrückstände einen intramolekularen Übergang von Ubiquitin auf den anderen Cysteinrückstand und von dort auf dessen Substrat bewirkt;
b) Bereitstellen einer Sonde mit einer Hydroxygruppe, welche in der Lage ist, mit C4520, C4572 und/oder der Mediator-Schleifenregion des aktiven MYCBP2, oder des Orthologs oder des Mutanten oder des Fragments davon zu interagieren; und
c) Detektieren, ob der Interaktionslevel der Sonde mit MYCBP2, dem Ortholog oder dem Mutanten oder Fragment davon im Vergleich zum Interaktionslevel bei Nichtvorliegen der Testsubstanz moduliert wird.

2. Verfahren nach Anspruch 1, wobei die Sonde ein nicht endogenes Substrat aus aktivem MYCBP2 umfasst.

3. Verfahren nach Anspruch 1 oder Anspruch 2, wobei der Modulator ein Inhibitor ist, und Schritt c) Detektieren umfasst, ob der Interaktionslevel der Sonde mit MYCBP2, oder dem Ortholog oder dem Mutanten oder Fragment davon im Vergleich zum Interaktionslevel bei Nichtvorliegen der Testsubstanz reduziert ist.

4. Verfahren nach einem der vorhergehenden Ansprüche, wobei die Sonde mit einer Oberfläche konjugiert ist.

5. Verfahren nach Anspruch 1, wobei die Sonde ein Peptid umfasst, wobei optionsweise die Sonde Threonin oder Serin umfasst.

6. Verfahren nach Anspruch 1, wobei die Probe ein kleines Molekül umfasst, welches ein Molekulargewicht von nicht über 2kDa aufweist.

7. Verfahren nach Anspruch 5 oder Anspruch 6, wobei die Sonde Tris, Glycerol oder HEPES umfasst.

8. Verfahren nach einem der vorhergehenden Ansprüche, wobei das Ortholog ausgewählt ist aus Maus-Phrl, Zebrafisch-Esrom/phr1, Drosophila Highwire und C.elegans RPM-1.

9. Verfahren nach einem der vorhergehenden Ansprüche, wobei die Testsubstanz ein Peptid, ein kleines Molekül, ein Aptamer, einen Antikörper oder ein Antikörperfragment umfasst.

10. Verfahren nach einem der vorhergehenden Ansprüche, wobei die Sonde ein Label umfasst.

11. Verfahren nach Anspruch 10, wobei das Label eine Affinitätsmarkierung umfasst.

12. Verfahren nach einem der vorhergehenden Ansprüche, wobei das Verfahren In-Kontakt-Bringen eines Fragmentes von MYCBP2 umfasst, wobei das Fragment Rückstände 4390 bis 4572 von MYCBP2 umfasst.

## Revendications

1. Procédé permettant d'identifier un modulateur de l'activité d'estérification de MYCBP2 vers des substrats contenant de l'hydroxy, le procédé comprenant :
a) la mise en contact de MYCBP2, ou d'un orthologue, ou d'un mutant, ou d'un fragment de celui-ci comprenant au moins le site actif de la protéine native de MYCBP2 avec une substance test, dans lequel le site actif comprend deux cystéines catalytiques, dans lequel les cystéines catalytiques sont C4520 et C4572 (la numérotation se faisant selon l'isoforme canonique de MYCBP2 pleine longueur native divulguée dans la description), dans lequel C4520 réside au sein d'une région de boucle médiatrice représentée par les résidus 4515 à 4531, dans lequel la transthiolation entre l'enzyme de conjugaison à E2 (E2) et l'un des résidus cystéine conduit à un relais intramoléculaire d'ubiquitine vers l'autre résidu cystéine et ensuite vers son substrat ;
b) la fourniture d'une sonde avec un groupe hydroxy qui est apte à interagir avec C4520, C4572 et/ou la région de boucle médiatrice du MYCBP2 actif, ou de l'orthologue, ou du mutant ou du fragment de celui-ci ; et
c) la détection du fait que le niveau d'interaction de la sonde avec le MYCBP2, l'orthologue, ou le mutant ou le fragment de celui-ci est modulé en comparaison avec le niveau d'interaction en l'absence de la substance test ou non.

2. Procédé selon la revendication 1, dans lequel la sonde comprend un substrat non endogène du MYCBP2 actif.

3. Procédé selon la revendication 1 ou la revendication 2, dans lequel le modulateur est un inhibiteur et l'étape c) comprend la détection du fait que le niveau d'interaction de la sonde avec le MYCBP2, ou l'orthologue, ou le mutant, ou le fragment de celui-ci est réduit en comparaison avec le niveau d'interaction en l'absence de la substance test ou non.

4. Procédé selon une quelconque revendication précédente, dans lequel la sonde est conjuguée à une surface.

5. Procédé selon la revendication 1, dans lequel la sonde comprend un peptide, facultativement, dans lequel la sonde comprend la thréonine ou la sérine.

6. Procédé selon la revendication 1, dans lequel la sonde comprend une petite molécule présentant un poids moléculaire ne dépassant pas 2 kDa.

7. Procédé selon la revendication 5 ou la revendication 6, dans lequel la sonde comprend du tris, du glycérol ou de l'HEPES.

8. Procédé selon une quelconque revendication précédente, dans lequel l'orthologue est sélectionné à partir du Phr1 de souris, de l'Esrom/phr1 de poisson zèbre, de Drosophila Highwire et de C.elegans RPM-1.

9. Procédé selon une quelconque revendication précédente, dans lequel la substance test comprend un peptide, une petite molécule, un aptamère, un anticorps ou un fragment d'anticorps.

10. Procédé selon une quelconque revendication précédente, dans lequel la sonde comprend un marqueur.

11. Procédé selon la revendication 10, dans lequel le marqueur comprend une étiquette d'affinité.

12. Procédé selon une quelconque revendication précédente, dans lequel le procédé comprend la mise en contact d'un fragment de MYCBP2, dans lequel le fragment comprend les résidus 4390 à 4572 de MYCBP2.
